(19) Europäisches Patentamt
European Patent Office
Office européen des brevets

(11) Veröffentlichungsnummer: **0 449 079 B1**

# (12) EUROPÄISCHE PATENTSCHRIFT

(45) Veröffentlichungstag der Patentschrift: **15.11.95**

(51) Int. Cl.⁶: **C07K 5/08**, C07K 5/10, C07K 7/06, A61K 38/04

(21) Anmeldenummer: **91104195.2**

(22) Anmeldetag: **19.03.91**

## (54) Hydantoinderivate.

(30) Priorität: **24.03.90 DE 4009506**

(43) Veröffentlichungstag der Anmeldung:
**02.10.91 Patentblatt 91/40**

(45) Bekanntmachung des Hinweises auf die Patenterteilung:
**15.11.95 Patentblatt 95/46**

(84) Benannte Vertragsstaaten:
**BE DE DK ES FR GB GR IT LU NL**

(56) Entgegenhaltungen:

**CHEMICAL ABSTRACTS, Band 103, Nr. 25, 23. Dezember 1985, Seiten 943-944,Zusammenfassung Nr. 215779s, Columbus, Ohio, US; B. SCHWENZER et al.: "Cleavage of Z-group and formation of hydantoin during alkaline saponification of Z-peptide esters", & J. PRAKT. CHEM. 1985, 327(3), 479-86**

(73) Patentinhaber: **CASSELLA Aktiengesellschaft**
**Hanauer Landstrasse 526**
**D-60386 Frankfurt (DE)**

(72) Erfinder: **König, Wolfgang, Dr.**
**Eppsteiner Strasse 25**
**W-6238 Hofheim am Taunus (DE)**
Erfinder: **Just, Melitta, Dr.**
**Theodor-Heuss-Strasse 80**
**W-6070 Langen (DE)**
Erfinder: **Jablonka, Bernd, Dr.**
**Dachbergstrasse 19 a**
**W-6232 Bade Soden am Taunus (DE)**

**EP 0 449 079 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Hydantoinderivate mit Thrombozytenaggregation-hemmender Wirkung.
Gegenstand der Erfindung sind Verbindungen der allgemeinen Formel I

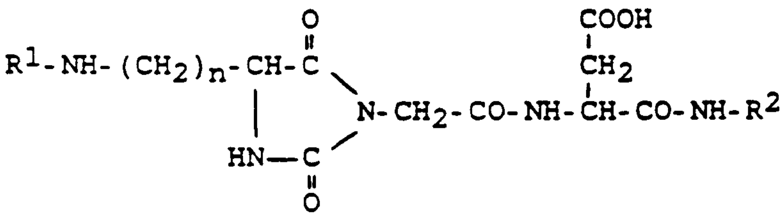

(I)

in welcher

- n für eine ganze Zahl 3 oder 4 steht;
- $R^1$ $C_1$-$C_6$-Alkyl oder einen Rest der Formel II bedeutet

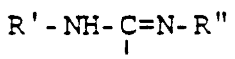

(II)

worin

- R' und R" unabhängig voneinander Wasserstoff oder $C_1$-$C_6$-Alkyl bedeuten;
- $R^2$ Wasserstoff, -NH-CO-$NH_2$ oder $C_1$-$C_{18}$-Alkyl bedeutet, das gegebenenfalls ein- oder mehrfach durch gleiche oder verschiedene Reste aus der Reihe Hydroxy, Carboxy, Carbamoyl, Carboxamido, Amino, Mercapto, $C_1$-$C_{18}$-Alkoxy, Guanidino, $C_3$-$C_8$-Cycloalkyl, Halogen, Nitro, Trifluormethyl und einen Rest $R^3$ substituiert ist, wobei
- $R^3$ für $C_6$-$C_{14}$-Aryl, $C_6$-$C_{14}$-Aryl-$C_1$-$C_8$-alkyl oder einen mono- oder bicyclischen 5- bis 12-gliedrigen heterocyclischen Ring, der aromatisch, teilhydriert oder vollständig hydriert sein kann und der als Heteroelement ein, zwei oder drei gleiche oder verschiedene Stickstoff-, Sauerstoff- oder Schwefel-Atome enthalten kann, steht, wobei der Aryl- und unabhängig voneinander der Heterocyclus-Rest gegebenenfalls ein- oder mehrfach durch gleiche oder verschiedene Reste aus der Reihe $C_1$-$C_{18}$-Alkyl, Hydroxy, $C_1$-$C_{18}$-Alkoxy, Halogen, Nitro und Trifluormethyl substituiert sind; oder für einen Rest $R^4$ steht; wobei
- $R^4$ $NR^5R^6$; $OR^5$; $SR^5$; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest bedeutet, bei dem die Peptidbindung zu NH-$CH_2$ reduziert sein kann, sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen Rest $COR^{4'}$, bedeutet worin $R^{4'}$ wie $R^4$ definiert ist;
- $R^5$ Wasserstoff, gegebenenfalls durch eine Aminogruppe substituiertes $C_1$-$C_{18}$-Alkyl, $C_6$-$C_{14}$-Aryl, $C_6$-$C_{14}$-Aryl-$C_1$-$C_8$-alkyl, $C_1$-$C_{18}$-Alkylcarbonyl, $C_1$-$C_{18}$-Alkyloxycarbonyl, $C_6$-$C_{14}$-Arylcarbonyl, $C_6$-$C_{12}$-Aryl-$C_1$-$C_8$-alkylcarbonyl, $C_6$-$C_{18}$-Aryl-$C_1$-$C_{18}$-alkyloxycarbonyl, einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest bedeutet, bei den die Peptidbindung zu NH-$CH_2$ reduziert sein kann;
- $R^6$ Wasserstoff, $C_1$-$C_{18}$-Alkyl, $C_6$-$C_{12}$-Aryl oder $C_6$–$C_{12}$-Aryl-$C_1$-$C_8$-alkyl bedeutet;

sowie deren physiologisch verträgliche Salze.

Alkyl kann geradkettig oder verzweigt sein. Entsprechendes gilt für davon abgeleitete Reste, wie z.B. Alkoxy, Alkanoyl und Aralkyl.

Unter Cycloalkyl werden auch Alkyl substituierte Reste, wie z.B. 4-Methylcyclohexyl oder 2,3-Dimethylcyclopentyl verstanden.

$C_6$-$C_{14}$-Aryl ist beispielsweise Phenyl, Naphthyl, Biphenylyl oder Fluorenyl; bevorzugt sind Phenyl und Naphthyl. Entsprechendes gilt auch für davon abgeleitete Reste, wie z.B. Aryloxy, Aroyl, Aralkyl und Aralkoxy. Unter Aralkyl versteht man z.B. einen mit $C_1$-$C_8$-Alkyl verknüpften unsubstituierten oder substitu-

ierten $C_6$-$C_{14}$-Arylrest, wie z.B. Benzyl, 1- und 2-Naphthylmethyl, Halobenzyl und Alkoxybenzyl, wobei Aralkyl jedoch nicht auf die genannten Reste beschränkt wäre.

Heterozyklen im Sinne vorstehender Definitionen sind beispielsweise Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindazolyl, Indazolyl, Phthalazinyl, Chinolyl, Isochinolyl, Chinoxalinyl, Chinazolinyl, Cinnolinyl oder ein benzanelliertes, cyclopenta-, cyclohexa- oder cyclohepta-anelliertes Derivat dieser Reste.

Diese Heterocyclen können an einem Stickstoffatom durch Oxide, $C_1$-$C_7$-Alkyl, z.B. Methyl oder Ethyl, Phenyl oder Phenyl-$C_1$-$C_4$-alkyl, z.B. Benzyl, und/oder an einem oder mehreren Kohlenstoffatomen durch $C_1$-$C_4$-Alkyl, z.B. Methyl, Phenyl, Phenyl-$C_1$-$C_4$-alkyl, z.B. Benzyl, Halogen, Hydroxy, $C_1$-$C_4$-Alkoxy, z.B. Methoxy, Phenyl-$C_1$-$C_4$-alkoxy, z.B. Benzyloxy, oder Oxo substituiert und teilweise oder vollständig gesättigt sein.

Derartige Reste sind beispielsweise 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, z.B. 1-Methyl-, 5-Methyl-, 5-Methoxy-, 5-Benzyloxy-, 5-Chlor- oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl. Teilhydrierte oder vollständig hydrierte heterozyklische Ringe sind beispielsweise Dihydropyridinyl, Pyrrolidinyl, z.B. 2-, 3- oder 4-N-methylpyrrolidinyl, Piperazinyl, Morpholino, Thiomorpholino, Tetrahydrothienyl, Benzodioxolanyl.

Halogen steht für Fluor, Chlor, Brom oder Jod, insbesondere für Fluor oder Chlor.

Natürliche oder unnatürliche Aminosäuren können, falls chiral, in der D- oder L-Form vorliegen. Bevorzugt sind $\alpha$-Aminosäuren. Beispielsweise seien genannt (vgl. Houben-Weyl, Methoden der organischen Chemie, Band XV/1 und 2, Stuttgart, 1974):

Aad, Abu, $\gamma$Abu, ABz, 2ABz, $\epsilon$Aca, Ach, Acp, Adpd, Ahb, Aib, $\beta$Aib, Ala, $\beta$Ala, $\Delta$Ala, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, hyp, 3Hyp, Ile, Ise, Iva, Kyn, Lant, Lcn, Leu, Lsg, Lys, $\beta$Lys, $\Delta$Lys, Met, Mim, Min, nArg, Nle, Nva, Oly, Orn, Pan, Pec, Pen, Phe, Phg, Pic, Pro, $\Delta$Pro, Pse, Pya, Pyr, Pza, Qin, Ros, Sar, Sec, Sem, Ser, Thi, $\beta$Thi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)-aminoessigsäure.

Unter Aminosäureseitenketten werden Seitenketten von natürlichen oder unnatürlichen Aminosäuren verstanden. Azaaminosäuren sind natürliche oder unnatürliche Aminosäuren, wobei der Zentralbaustein -CHR- bzw. $-CH_2$-durch -NR- bzw. -NH- ersetzt ist.

Als Rest einer Iminosäure kommen insbesondere Reste von Heterocyclen aus der folgenden Gruppe in Betracht:

Pyrrolidin-2-carbonsäure; Piperidin-2-carbonsäure; Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Aza-bicyclo[2.2.2]octan-3-carbonsäure; 2-Azabicyclo[2.2.1]-heptan-3-carbonsäure; 2-Azabicyclo[3.1.0]hexan-3-carbonsäure; 2-Azaspiro[4.4]nonan-3-carbonsäure; 2-Azaspiro[4.5]-decan-3-carbonsäure; Spiro[(bicyclo[2.2.1]-heptan)-2,3-pyrrolidin-5-carbonsäure; Spiro[-(bicyclo[2.2.2]octan)-2,3-pyrrolidin-5-carbonsäure; 2-Azatricyclo[4.3.0.1$^{6,9}$]decan-3-carbonsäure; Decahydrocyclohepta[b]pyrrol-2-carbonsäure; Decahydrocycloocta[b]pyrrol-2-carbonsäure; Octahydrocyclopenta[c]-pyrrol; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure; Hydroxyprolin-2-carbonsäure; die alle gegebenenfalls substituiert sein können:

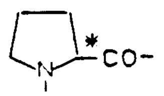 ; 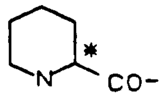 ;

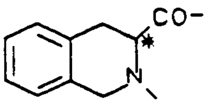 ; 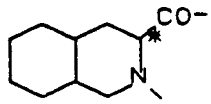 ; 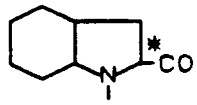 ; 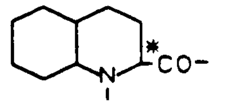 ;

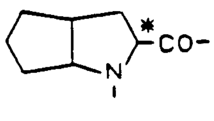 ; 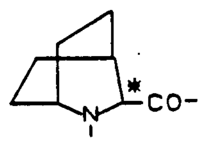 ; 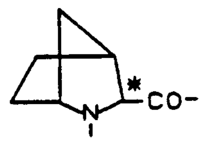 ; 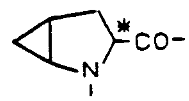 ;

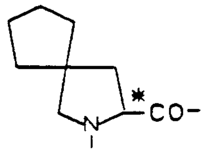 ; 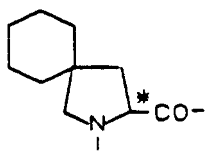 ; 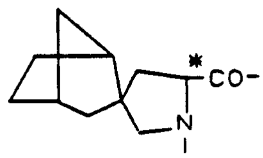 ;

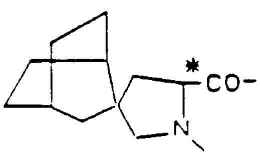 ; 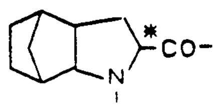 ; 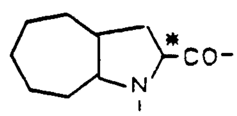 ;

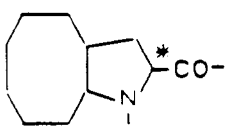 ; 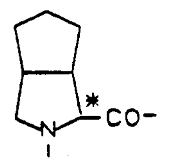 ; 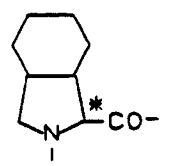 ;

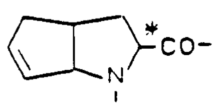 ; 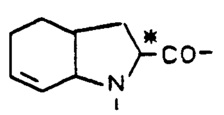 ; 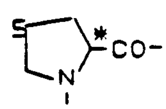 ;

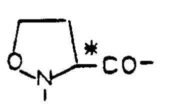 ; 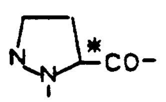 ; 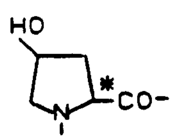 ;

Die oben genannten Reste zugrundeliegenden Heterocyclen sind beispielsweise bekannt aus US-A 4 344 949, US-A 4 374 847, US-A 4 350 704, EP-A 50 800, EP-A 31 741, EP-A 51 020, EP-A 49 658, EP-A 49 605, EP-A 29 488, EP-A 46 953, EP-A 52 870, EP-A 271 865, DE-A 32 26 768, DE-A 31 51 690, DE-A 32 10 496, DE-A 32 11 397, DE-A 32 11 676, DE-A 32 27 055, DE-A 32 42 151, DE-A 32 46 503 und DE-A 32 46 757.

Ferner werden einige dieser Heterocyclen vorgeschlagen in DE-A 38 18 850.3.

Dipeptide können als Bausteine natürliche oder unnatürliche Aminosäuren, Iminosäuren sowie Azaaminosäuren enthalten. Ferner können die natürlichen oder unnatürlichen Aminosäuren, Iminosäuren, Azaaminosauren und Dipeptide auch als Ester bzw. Amide vorliegen, wie z.B. Methylester, Ethylamid, Semicarbazid, $\omega$-Amino-$C_4$-$C_8$-alkylamid.

Funktionelle Gruppen der Aminosäuren, Iminosäuren und Dipeptide können geschützt vorliegen. Geeignete Schutzgruppen wie z.B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt:
Aloc, Pyoc, Fmoc, Tcboc, Z, Boc, Ddz, Bpoc, Adoc, Msc, Moc, $Z(NO_2)$, $Z(Hal_n)$, Bobz, Iboc, Adpoc, Mboc,

Acm, tert.-Butyl, OBzl, ONbzl, OMbzl, Bzl, Mob, Pic, Trt.

Unter Salzen von Verbindungen der Formel (I) sind insbesondere pharmazeutisch verwendbare oder nicht-toxische Salze zu verstehen.

Solche Salze werden beispielsweise von Verbindungen der Formel (I), welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin und Tris-(2-hydroxy-ethyl)-amin.

Verbindungen der Formel (I), welche basische Gruppen, z.B. eine Aminogruppe oder ein Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I

worin

- $R^2$ Wasserstoff oder $C_1$-$C_8$-Alkyl bedeutet, das gegebenenfalls 1- bis 4-fach durch gleiche oder verschiedene Reste aus der Reihe Hydroxy, $C_1$-$C_6$-Alkoxy,Amino, Carboxy, Carbamoyl, Guanidino, $C_3$-$C_6$-Cycloalkyl, Halogen und einen Rest $R^3$ substituiert ist, wobei
- $R^3$ für $C_6$-$C_{12}$-Aryl, $C_6$-$C_{12}$-Aryl-$C_1$-$C_4$-alkyl oder einen mono- oder bicyclischen 5- bis 12-gliedrigen heterocyclischen, aromatischen Ring, steht, der als Heteroelement ein oder zwei Stickstoffatome enthält, wobei unabhängig voneinander der Aryl- und Heterocyclus-Rest gegebenenfalls ein-, zwei- oder dreifach durch gleiche oder verschiedene Reste aus der Reihe $C_1$-$C_6$-Alkyl, $C_1$-$C_6$-Alkoxy, Halogen, Nitro, Hydroxy und Trifluormethyl substituiert sind; oder für einen Rest $R^4$ steht, wobei
- $R^4$ $NR^5R^6$; $OR^5$; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen Rest $COR^{4'}$ bedeutet, worin $R^{4'}$ wie $R^4$ definiert ist;
- $R^5$ und $R^6$ unabhängig voneinander Wasserstoff, $C_1$-$C_8$-Alkyl oder $C_6$-$C_{12}$-Aryl bedeutet.

Insbesondere seien Verbindungen der Formel I genannt, worin

- $R^1$ ein Rest der Formel II bedeutet, worin
- R' und R'' unabhängig voneinander Wasserstoff oder $C_1$-$C_2$-Alkyl bedeuten;
- $R^2$ Wasserstoff oder $C_1$-$C_6$-Alkyl bedeutet, das zweifach durch gleiche oder verschiedene Reste $R^4$ substituiert ist, wobei
- $R^4$ Hydroxy; Amino; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest bedeutet, bei dem die Peptidbindung zu $NH$-$CH_2$ reduziert sein kann, sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen Rest $COR^{4'}$ bedeutet, worin $R^{4'}$ wie $R^4$ definiert ist, oder

  Verbindungen der Formel I, worin
- $R^2$ Methyl bedeutet, das zweifach durch verschiedene Reste $R^4$ und $COR^{4'}$ substituiert ist, wobei $R^4$ bzw. $R^{4'}$ wie oben beschrieben definiert sind.

Ganz besonders bevorzugt sind Verbindungen der Formel I, worin

- $R^1$ einen Rest der Formel II bedeutet, worin
- R' und R'' Wasserstoff bedeuten
- $R^2$ Wasserstoff oder $C_1$-$C_6$-Alkyl bedeutet, das ein- oder zweifach, unabhängig voneinander durch -OH, $NH_2$, -COOH, -$CONH_2$, -NH-C($NH_2$)=NH , $C_5$-$C_8$-Cycloalkyl, einen Rest $R^3$, wobei
- $R^3$ für $C_6$-$C_{14}$-Aryl, dau gegebenenfalls durch Hydroxy substituiert sein kann, einen bizyklischen 8- bis 12-gliedrigen heterozyklischen, aromatischen Ring, der als Heteroelement ein Stickstoffatom enthält, steht,

  oder einen Rest $R^4$ substituiert ist, wobei
- $R^4$ für $NR^5R^6$, einen natürlichen oder unnatürlichen Aminosäure-, Iminosäure-, gegebenenfalls N-$C_1$-$C_4$-alkylierten oder N-$C_6$-$C_{14}$-aryl-$C_1$-$C_4$-alkylierten Azaaminosäure- oder Dipeptid-Rest sowie deren Amide, oder einen Rest $COR^{4'}$, worin $R^{4'}$ wie $R^4$ definiert ist, steht,
- $R^5$ Wasserstoff,
- $R^6$ $C_1$-$C_6$-Alkyl bedeuten, und

n 3 bedeutet.

Hydantoine entstehen ganz allgemein durch basische Behandlung von Alkoxycarbonyl-oder Aralkoxycarbonyl-Peptiden der allgemeinen Formel III [J.S. Fruton und M. Bergmann, J. Biol. Chem. 145 (1942) 253-265; C.A. Dekker, S.P. Taylor, Jr. und J.S. Fruton, J. Biol. Chem. 180 (1949) 155-173; M.E. Cox, H.G. Garg, J. Hollowood, J.M. Hugo, P.M. Scopes und G.T. Young, J. Chem. Soc. (1965) 6806-6813; W. Voelter und A. Altenburg, Liebigs Ann. Chem. (1983) 1641-1655]:

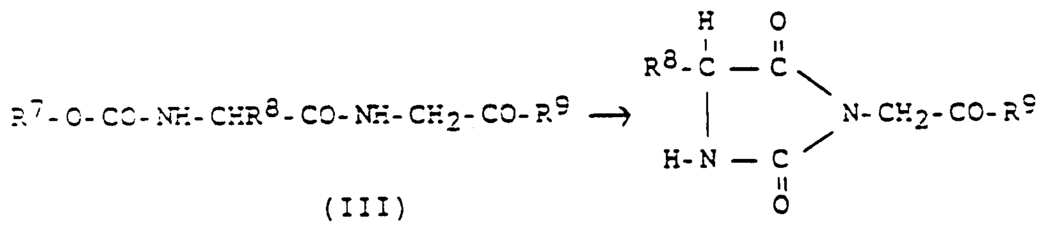

(III)

worin $R^7$ Benzyl oder tert. Butyl, $R^8$ eine beliebige Aminosäureseitenkette und $R^9$ ein Amid. einen Aminosäure- oder einen Peptid-Rest bedeuten. Dabei racemisiert jedoch die N-terminale Aminosäure [W. Voelter und A. Altenburg, Liebigs Ann. Chem. (1983) 1641-1655].

Eine milde Methode ist dagegen die Zyklisierung zu den Hydantoinen aus Verbindungen der Formel III unter neutralen Bedingungen durch Behandlung mit Tetrabutylammoniumfluorid in Tetrahydrofuran unter Rückfluß) [J. Pless, J. Org. Chem. 39 (1974) 2644-2646].

Eine weitere Möglichkeit einer milden Zyklisierung ist die Trimethylsilylierung der Peptidbindung zwischen der N-terminalen Aminosäure und dem benachbarten Glycin mit Bistrimethylsilyltrifluoroacetamid in Acetonitril (4 Stunden unter Rückfluß) [J.S. Davies, R.K. Merritt und R.C. Treadgold, J. Chem. Soc. Perkin Trans. I (1982) 2939-2947].

Überraschenderweise wurde nun gefunden, daß Peptide der Formel IIIa nach längerer Zeit bereits bei Raumtemperatur oder mit Tetrahydrofuran kurz im Rückfluß gekocht, zu den Hydantoin-Derivaten zykliaieren.

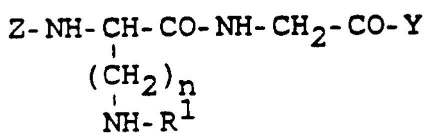

(IIIa)

wobei
Z Benzyloxycarbonyl und
Y OtBu, Asp(OtBu)-NH-$R^2$, wobei mögliche Carboxylgruppen als Ester vorliegen sollen, bedeuten und n, $R^1$ und $R^2$ wie oben beschrieben definiert sind.

Durch Kondensation von Nitroarginin oder Nitrohomoarginin mit Isocyanato-essigsäureethylester erhält man Harnstoffderivate, die durch Erhitzen in Salzsäure unter Verseifung des Esters zu Hydantoinderivaten der Formel VIa cyclisieren (K. Schlögl und H. Fabitschowitz, Monatsh. Chem. 84 (1953), 937):

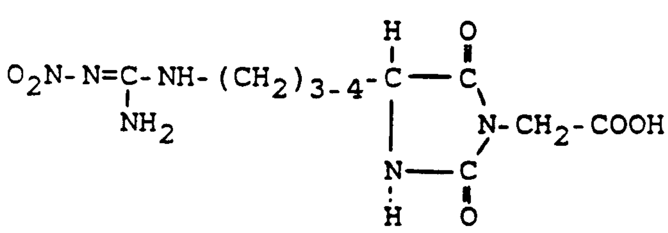

(VIa)

Ein weiterer Gegenstand der Erfindung ist somit die Herstellung der Verbindungen der Formel I, dadurch gekennzeichnet, daß man

$a_1$) Verbindungen der Formel IV,

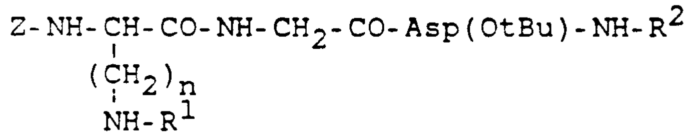

(IV)

in der Z, $R^1$, $R^2$ und n die oben beschriebenen Bedeutungen haben, in Tetrahydrofuran ca. 2-3 Stunden im Rückfluß erhitzt oder

$a_2$) die Verbindungen der allgemeinen Formel VI und VII

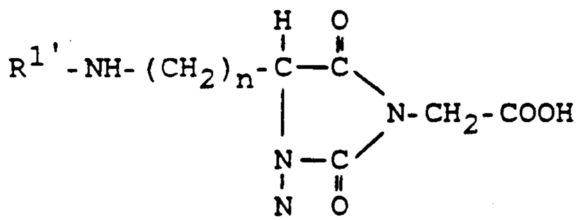

+ $H\text{-}Asp(OX)\text{-}NH\text{-}R^2$

(VI) (VII)

worin

$R^{1'}$ $-C(NH_2)=N\text{-}NO_2$ bedeutet oder wie $R^1$ oben definiert ist,

X für tBu oder Bzl steht und $R^2$ und n die oben beschriebenen Bedeutungen haben,

mittels der allgemeinen Methoden der Peptidchemie kondensiert, und

b) die so entstandenen Hydantoine der allgemeinen Formel V

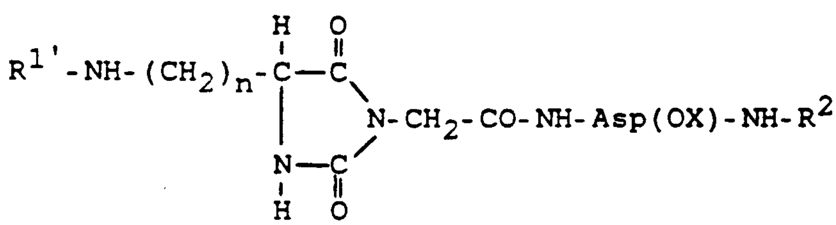

(V)

durch katalytische Hydrierung und/oder saure Abspaltung der Schutzgruppen in die erfindungsgemäßen Verbindungen der allgemeinen Formel I überführt.

Die Verbindungen der Formel VI sind neu. Sie werden aus den Verbindungen der allgemeinen Formel IIIa (Y = OtBu) durch Erhitzen in Tetrahydrofuran und anschließender Behandlung mit Trifluoressigsäure oder wie bei der Herstellung der Verbindungen der Formel VIa beschrieben gewonnen.

Die Ausgangspeptide der Formel IV und VII werden in der Regel vom C-terminalen Ende her stufenweise aufgebaut. Die Peptidknüpfungen können mit den literaturbekannten Kupplungsmethoden der Peptidchemie durchgeführt werden z.B. Houben-Weyl, Methoden der organischen Chemie, Band 15/2; B. Merrifield J. Am. Chem. Soc. 85 (1963) 2149; R. Sheppard, Int. J. Peptide Protein Res. 21 (1983) 118).

Die erfindungsgemäBen Verbindungen der Formel I haben die Fähigkeit die Zell-Zell-Adhäsion zu hemmen, die auf einer Interaktion von Arg-Gly-Asp-enthaltenden Glykoproteinen mit den sogenannten Integrinen beruht. Integrine sind Transmembran-Glykoproteine, Rezeptoren für Arg-Gly-Asp-enthaltenden Zellmatrix-Glykoproteine [E. Ruoslahti und M.D. Pierschbachcr, Science 238 (1987) 491-497; D.R. Phillips, I.F. Charo, L.V. Parise und L.A. Fitzgerald, Blood 71 (1988) 831-843].

Die erfindungsgemäßen neuen Hydantoin-Derivate der Formel I hemmen die Thrombozytenaggregation, die Metastasierung sowie die Osteoclastenbindung an die Knochenoberflächen.

Eine akute Anwendung finden die Hydantoin-Derivate der Formel I daher bei Thrombosegefahr und der Gefahr einer Reocclusion bei Herzinfarkt; eine chronische Anwendung bei der Prävention der Arteriosklerose. Weitere Anwendung ist während Krebsoperationen und auch prophylaktisch bei Krebs gegeben. Ferner kann Osteoporose durch Hemmung der Osteoclastenbindung an die Knochenoberflächen vermieden werden.

Ein Vorteil der erfindungsgemäßen Hydantoin-Verbindungen gegenüber den bisher bekannten Arg-Gly-Asp-enthaltenden Peptiden [M.D. Pirschbacher und E. Ruoslahti, Nature 309 (1984) 30-33; M.D. Pirschbacher und E. Ruoslahti, J. Biol. Chem. 262 (1987) 17294-17298; H.M. Charon et al., Amer. Peptide Symp. 1989; S.P. Adams et al., Amer. Peptide Symp. 1989; EP-A2 341 915] ist neben der guten Wirkungsstärke vor allem die größere enzymatische Stabilität und die längere Halbwertszeit.

Geprüft werden die Verbindung vor allem auf ihre hemmende Wirkung bei der Blutplättchenaggregation und der Anhaftung von Fibrinogen an Blutplättchen. Verwendet werden gelfiltrierte Blutplättchen aus human Spendeblut, die mit ADP oder Thrombin aktiviert werden.

Hemmung der Thrombozytenaggregation an mit Adenosindiphouphat (ADP) oder Thrombin (THR) stimulierten human GFP (gel filtered platelets) (Marguerie, Plow and Edgington, J. Biol. Chem. 254 (1979) 5357-5363)

| | IC50 (µM) | |
|---|---|---|
| | ADP | THR |
| Z-D-Arg-Gly-Asp-NH-Mbh | 20 | 20 |
| H-D-Arg-Gly-Asp-NH-Mbh | 50 | 30 |
| CO-D-Arg-Gly-Asp-NH-Mbh | 4 | 3 |
| Z-D-Arg-Gly-Asp-$NH_2$ | 100 | 80 |
| H-D-Arg-Gly-Asp-$NH_2$ | 500 | 100 |
| CO-D-Arg-Gly-Asp-$NH_2$ | 20 | 15 |
| H-Arg-Gly-Asp-NH-Mbh | 100 | 80 |
| CO-Arg-Gly-Asp-NH-Mbh | 10 | 4 |
| H-Arg-Gly-Asp-$NH_2$ | 500 | 200 |
| CO-Arg-Gly-Asp-$NH_2$ | 40 | 40 |
| CO-Arg-Gly-Asp-Phe-OH | 10 | 3 |
| CO-Arg-Gly-Asp-Arg-Trp-$NH_2$ | 10 | 7 |
| CO-Arg-Gly-Asp-Val-OH | 1 | 0,5 |
| CO-Arg-Gly-Asp-Val-$NH_2$ | 10 | 3 |
| CO-Arg-Gly-Asp-Arg-Val-$NH_2$ | 9 | 4,5 |
| CO-Arg-Gly-Asp-Arg-isobutylamid | 5,5 | 2,5 |
| CO-Arg-Gly-Asp-Phe-Azagly-$NH_2$ | 30 | 10 |
| CO-Arg-Gly-Asp-Phe-N($CH_3$)-NH-$CONH_2$ | 60 | 20 |
| CO-Arg-Gly-Asp-Phe-N(2-naphthylmethyl)-NH-$CONH_2$ | 15 | 7 |
| CO-Arg-Gly-Asp-Gln-OH | 3 | 1,5 |
| CO-Arg-Gly-Asp-Arg-4-Abu-OH | 15 | 4 |
| CO-Arg-Gly-Asp-Ser-OH | 10 | 4 |
| CO-Arg-Gly-Asp-Arg-Hyp-NH-$C_2H_5$ | 3 | 4 |

CO-Arg-Gly- = 5-(S)-(3-Guanidino-propyl)-2,4-dioxo-imidazolidin-3-yl)-acetyl-

Mbh = 4,4'-Dimethoxybenzhydryl-

Abkürzungen:

| | |
|---|---|
| Acm | Acetamidomethyl |
| Adoc | 1-Adamantyloxycarbonyl |
| Adpoc | 1-(1-Adamantyl)-1-methyl-ethoxycarbonyl |
| Aloc | Allyloxycarbonyl |
| Boc | tert.-Butyloxycarbonyl |
| Bpoc | 1-(4-biphenylyl)-1-methyl-ethoxycarbonyl |
| Cha | Cyclohexylalanin |
| Chg | Cyclohexylglycin |
| DCC | Dicyclohexylcarbodiimid |
| Ddz | α,α-Dimethyl-3,5-dimethoxybenzyloxycarbonyl |
| Dobz | 4-Dihydroxyborylbenzyloxycarbonyl |
| Fmoc | 9-Fluorenylmethyloxycarbonyl |
| HOBt | 1-Hydroxybenzotriazol |
| HOObt | 3-Hydroxy-4-oxo-3,4-dihydro-1,2,3-benzotriazin |
| Ibcc | Isobornyloxycarbonyl |
| Mboc | 1-Methyl-cyclobutyloxycarbonyl |
| Moc | 4-Methoxybenzyloxycarbonyl |
| Msc | Methylsulfonylethyloxycarbonyl |
| Npg | Neopentylglycin |
| Pyoc | 4-Pyridylmethyloxycarbonyl |
| Tbg | Tert.-butylglycin |
| Tcboc | 2,2,2-Trichloro-tert.-butyloxycarbonyl |
| Thia | 2-Thienylalanin |
| Z | Benzyloxycarbonyl |
| $Z(Hal_n)$ | halogensubstituiertes Benzyloxycarbonyl |
| $Z(NO_2)$ | 4-Nitro-benzyloxycarbonyl |

**Beispiele**

Aminosäureanalyse: Hydrolyse in 6N HCl (24 Stunden bei 120 °C). Der Gehalt von Arg und Gly ist bei den Hydantoin-Derivaten stark reduziert (B. Schwenzer, E. Weber und G. Losse, J. Prakt. Chem. 327 (1985) 479-486).

1. [5-(3-Guanidino-propyl)-2,4-dioxo-imidazolidin-3-yl] acetyl-L-asparaginsäure-4,4'-dimethoxybenzhydrylamid und [5-(3-Guanidino-propyl)-2,4-dioxo-imidazolidin-3-yl] acetyl-L-asparaginsäure-amid.

**1a. Z-Asp(OtBu)-4,4'-dimethoxybenzhydrylamid**

Zu einer Lösung von 6,5 g (20 mmol) Z-Asp(OtBu)-OH, 5,6 g (20 mmol) 4,4'-Dimethoxybenzhydrylamin-hydrochlorid und 2,7 g 1-Hydroxybenzotriazol (HOBt) in 30 ml Dimethylacetamid gab man bei 0 °C 2,6

ml N-Ethylmorpholin und 4,4 g Dicyclohexylcarbodiimid (DCC). Man rührte 1 Std. bei 0°C und ließ anschließend über Nacht bei Raumtemperatur stehen. Der Niederschlag wurde abgesaugt und das Filtrat eingeengt. Der Rückstand wurde zwischen Wasser und Essigester verteilt. Die organische Phase wurde nacheinander mit gesättigter $NaHCO_3$-Lösung, $KHSO_4/K_2SO_4$-Puffer, gesättigter $NaHCO_3$-Lösung und Wasser gewaschen und anschließend über $Na_2SO_4$ getrocknet und eingeengt. Der Rückstand wurde mit Petrolether verrieben, abgesaugt und im Vakuum getrocknet.
Ausb. 10 g.

Zur weiteren Reinigung wurde die Substanz in 35 ml Isopropanol heiß gelöst und mit Petrolether versetzt. Man ließ abkühlen, saugte ab und trocknete im Vakuum.
Ausb. 8,7 g;
Schmp. 126-127°, $[\alpha]_D^{20}$ = -1,0° (c=1, in Dimethylformamid).

**1b. H-Asp(OtBu)-4,4'-dimethoxybenzhydrylamid-hydrochlorid**

8,5 g (15,5 mmol) Z-Asp(OtBu)-4,4'-dimethoxybenzhydrylamid wurden in 400 ml Methanol suspendiert und an der Autobürette bei pH 4,5 unter Zugabe von methanolischer Salzsäure katalytisch (Pd auf $BaSO_4$) hydriert. Nach beendigter Hydrierung wurde der Katalysator abgesaugt und das Filtrat eingeengt. Der Rückstand wurde mit Petrolether verrieben, abgesaugt und getrocknet.
Ausb. 6,89 g.

Eine kleine Probe (980 mg) wurde zur Reinigung in 100 ml Wasser gelöst. Von Unlöslichem wurde filtriert und die klare Lösung gefriergetrocknet.
Ausb. 950 mg;
$[\alpha]_D^{20}$ = + 2,2° (c = 1, in Wasser).

**1c. Z-Gly-Asp(OtBu)-4,4'-dimethoxybenzhydrylamid**

Zu einer Lösung von 4,2 g (9,3 mmol) ungereinigtem H-Asp (OtBu)-4,4'-dimethoxybenzhydrylamid-hydrochlorid und 1,21 ml N-Ethylmorpholin in 50 ml Dimethylformamid gab man 3,3 g Z-Gly-OObt zu, rührte bis alles gelöst war und ließ über Nacht bei Raumtemperatur stehen. Die Lösung wurde im Vakuum eingeengt und der Rückstand wie in Versuch 1a beschrieben aufgearbeitet.
Ausb. 4,83 g (85,5 %);
$[\alpha]_D^{21}$ = -13,7° (c = 1, in Methanol).

**1d. H-Gly-Asp(OtBu)-4,4'-dimethoxybenzhydrylamid-hydrochlorid**

4,7 g (7,75 mmol) Z-Gly-Asp(OtBu)-4,4'-dimethoxybenzhydrylamid wurden wie in Beispiel 1b beschrieben katalytisch hydriert. Der Rückstand wurde mit Ether verrieben.
Ausb. 3,84 g.

Eine kleine Probe (800 mg) wurde wie in Beispiel 1b beschrieben gereinigt.
Ausb. 779 mg; $[\alpha]_D^{21}$ = -24,8° (c = 1, in Wasser).

**1e. Z-D-Arg-Gly-Asp(OtBu)-4,4'-dimethoxybenzhydrylamid**

Zu einer Lösung von 0,93 g (3 mmol) Z-D-Arg-OH, 1,52 g (3 mmol) H-Gly-Asp(OtBu)-4,4'-dimethoxybenzhydrylamid-hydrochlorid und 0,41 g HOBt in 10 ml Dimethylformamid gab man bei 0°C 0,66 9 DCC. Man ließ 1 Stunde bei 0°C rühren und anschließend bei Raumtemperatur über Nacht stehen. Der Niederschlag wurde abgesaugt und das Filtrat eingeengt. Der Rückstand wurde zwischen n-Pentanol und halbgesättigter $NaHCO_3$-Lösung verteilt. Es wurde noch 3 mal mit $NaHCO_3$-Lösung und einmal mit Wasser ausgeschüttelt und die organische Phase eingeengt. Der Rückstand wurde mit Petrolether verrieben, abgesaugt und getrocknet.
Ausb. 1,45 g. Die Substanz wurde ohne Reinigung zur nächsten Stufe verwendet.

**1f. [5-(R)-(3-Guanidino-propyl)-2,4-dioxo-imidazolin-3-yl]-acetyl-Asp-4,4'-dimethoxybenzhydrylamid und [5-(R)-(3-Guanidino-propyl)-2,4-dioxo-imidazolidin-3-yl]acetyl-L-Asp-amid.**

15,5 g Z-D-Arg-Gly-Asp(OtBu)-NH-Mbh werden in 775 ml absolutem Tetrahydrofuran 2 Stunden am Rückfluß gekocht. Anschließend wird eingeengt und der Rückstand im Hochvakuum getrocknet. Ausb. 14,6 g. Der obengewonnene Rückstand wird in 75 ml Methylenchlorid gelöst. Dazu gibt man 75 ml Trifluoressigsäure und läßt 30 Minuten bei Raumtemperatur stehen. Man engt ein. Der Rückstand wird mit Äther verrieben, abgesaugt und getrocknet.
Ausb. 12,2 g.

6,1 g der oben gewonnenen Rohsubstanz werden auf ®Sephadex LH 20 chromatographiert (Säule: 4 x 200 cm; Elutionsmittel: 0,5 M Essigsäure:Methanol wie 7,5:6).

Fraktion X.5-XIII.6: 540 mg. Massenspektrum (M + 1-peak bei 372) und Aminosäureanalyse (Asp 1,00, Gly 0,40, Arg 0,35; Gehalt an Peptid 86,5 %) deuten darauf hin, daß diese Fraktion [5-(3-Guanidinopropyl)-2,4-dioxo-imidazolidin-3-yl]acetyl-Asp-$NH_2$ ist.

Fraktion XIX.5-XXIII.6: 2,86 g. Massenspektrum (M + 1-peak bei 598) und Aminosäureanalyse (Asp 0,99, Gly 0,38, Arg 0,34; Gehalt an Peptid 88 %) deuten darauf hin, daß diese Fraktion [5-(3-Guanidinopropyl)-2,4-dioxo-imidazolidin-3-yl]acetyl-Asp-4,4'-dimethoxybenzhydrylamid ist.

**Beispiel 2**

**[5-(S)-(3-Guanidino-propyl)-2,4-dioxo-imidazolidin-3-yl] acetyl-Asp-4,4'-dimethoxybenzhydrylamid und [5-(S)-(3-Guanidinopropyl)-2,4-dioxo-imidazolidin-3-yl]acetyl-Asp-amid**

**2a. Z-Arg-Gly-Asp(OtBu)-4,4'-dimethoxybenzhydrylamid**

1,54 g Z-Arg-OH und 2,54 g HCl• H-Gly-Asp(OtBu)-NH-Mbh werden analog Beispiel 1e mit 675 mg HOBt und 1,1 g DCC in 25 ml Dimethylformamid umgesetzt und aufgearbeitet. Ausb. 4,2 g Rohsubstanz, $[\alpha]^{21}$ = -18° (c = 1 in Methanol).

**2b. [5-(S)-(3-Guanidino-propyl)-2,4-dioxo-imidazolidin-3-yl]acetyl-Asp-4,4'-dimethoxybenzhydrylamid und [5-(S)-(3-Guanidinopropyl)-2,4-dioxo-imidazolidin-3-yl]acetyl-Asp-amid**

Analog Beispiel 1f werden 3,65 g Z-Arg-Gly-Asp(OtBu)-4,4'-dimethoxybenzhydrylamid in 170 ml absolutem Tetrahydrofuran im Rückfluß erhitzt und anschließend in Methylenchlorid/Trifluoressigsäure die Schutzgruppen abgespalten. Ausb. 3,15 g. Die so gewonnene Substanz wird wie in Beispiel 1f beschrieben gereinigt:
Fraktion III.3-IV.7: 485 mg. Massenspektrum (M + 1-peak bei 372) und Aminosäureanalyse (Asp 0,99, Gly 0,36, Arg 0,37; Peptidgehalt: 60 %) deuten darauf hin, daß diese Fraktion [5-(S)-(3-Guanidinopropyl)-2,4-dioxo-imidazolidin-3-yl] acetyl-Asp-amid ist.

Fraktion VI.7-VII.7: 1,54 g. Massenspektrum (M + 1-peak bei 598) und Aminosäureanalyse (Asp 0,99, Gly 0,36, Arg 0,3; Peptidgehalt: 78 %) deuten darauf hin, daß diese Fraktion [5-(S)-(3-Guanidinopropyl)-2,4-dioxo-imidazolidin-3-yl] acetyl-Asp-4,4'-dimethoxybenzhydrylamid ist.

**Beispiel 3**

**[5-(S)-(3-Guanidino-propyl)-2,4-dioxo-imidazolidin-3-yl] acetyl-Asp-Phe-OH**

**3a. Z-Arg-Gly-OtBu**

Zu einer Lösung von 55 g Z-Arg-OH, 30,18 g HCl• H-Gly-OtBu und 24,3 g HOBt in 400 ml Dimethylformamid gibt man bei 0°C 39,6 g DCC. Man läßt 1 Stunde bei 0°C und 3 Stunden bei Raumtemperatur rühren und über Nacht bei Raumtemperatur stehen. Der DC-Harnstoff wird abgesaugt und das Filtrat eingeengt. Der Rückstand wird zwischen Essigester und gesättigter $NaHCO_3$-Lösung gegenstromverteilt (je 400 ml). Hierbei fällt nach der 3. Stufe die gewünschte Substanz im 1. Scheidetrichter aus.
Ausb. 40,2 g.
Die Mutterlauge und die übrigen Essigesterphasen wurden zusammen eingeengt und nochmals der Gegenstromverteilung zwischen Essigester und $NaHCO_3$ unterworfen. Wieder nach der 3. Stufe fiel die gewünschte Substanz im 1. Scheidetrichter aus. Ausb. 27,8 g.
Gesamtausbeute: 68 g (89,6 %), Schmp. 112-116°C unter Zersetzung.

**3b. [5-(S)-(3-Guanidino-propyl)-2,4-dioxo-imidazolidin-3-yl]essigsäure**

67,5 g Z-Arg-Gly-OtBu werden in 800 ml absolutem Tetrahydrofuran suspendiert und 2 Stunden im Rückfluß gekocht. Danach wird eingeengt und der Rückstand mit Methyl-tert.-butyl-ether verrieben. Der

Niederschlag wird abgesaugt und getrocknet. Ausb. 54,2 g.

53,5 g der oben gewonnenen Substanz werden in 535 ml 90 %iger wäßriger Trifluoressigsäure gelöst. Man läßt 1 Stunde bei Raumtemperatur stehen und engt ein. Der Rückstand wird mit Diethylether verrieben, abgesaugt und getrocknet. Ausb. 53 g [5-(S)-(3-Guanidino-propyl)-2,4-dioxoimidazolidin-3-yl]-essigsäure-trifluoracetat.

Zur Entfernung der Trifluoressigsäure wird oben gewonnene Substanz über 800 ml schwach basischen Ionenaustauscher (IRA-93) in Wasser bei 50 °C chromatographiert. Die Substanz fällt größtenteils beim Abkühlen aus dem Eluat kristallin aus. Ausb. 24,9 g, Schmp. 287-292 °; $[\alpha]_D^{21}$ = 1,0 ° (c = 1, in Eisessig).

**3c. [5-(S)-(3-Guanidino-propyl)-2,4-dioxo-imidazolidin-3-yl]acetyl-Asp(OtBu)-Phe-OtBu**

Zu einer Lösung von 1,29 g HCl•H-Asp(OtBu)-Phe-OtBu, 0,772 g [5-(S)-(3-Guanidino-propyl)-2,4-dioxo-imidazolidin-3-yl]essigsäure und 0,41 g HOBt in 30 ml Dimethylformamid gibt man bei 0 °C 0,66 g DCC und läßt 1 Stunde bei 0 °C rühren und über Nacht bei Raumtemperatur stehen. Der Niederschlag wird abgesaugt und das Filtrat eingeengt. Der Rückstand wird mit Diethylether verrieben und der Rückstand an ®Sephadex LH20 chromatographiert (Säule 4 x 200 cm; Elutionsmittel: Eicessig: n-Butanol:Wasser wie 3,5:4,3:43).
Ausb. 800 mg, $[\alpha]_D^{22}$ = -25,1 ° (c = 1, in Methanol)

**3d. [5-(S)-(3-Guanidino-propyl)-2,4-dioxo-imidazolidin-3-yl]acetyl-Asp-Phe-OH**

610 mg [5-(R)-(3-Guanidino-propyl)-2,4-dioxo-imidazolidin-3-yl]acetyl-Asp(OtBu)-Phe-OtBu werden in 5 ml 90 %iger wäßriger Trifluoressigsäure gelöst. Man läßt 1 Stunde bei Raumtemperatur stehen und engt ein. Der Rückstand wird zwischen Wasser und Diethylether ausgeschüttelt und gefriergetrocknet. Ausb. 490 mg, $[\alpha]_D^{23}$ = -18,2 ° (c = 1, in Wasser)

**Beispiel 4**

**[5-(S)-(3-Guanidino-propyl)-2,4-dioxo-imidazolidin-3-yl] acetyl-Asp-Arg-Trp-$NH_2$**

Analog Beispiel 3c werden 0,772 g [5-(S)-(3-Guanidino-propyl)-2,4-dioxo-imidazolidin-3-yl]essigsäure und 2,625 g H-Asp(OtBu)-Arg-Trp-$NH_2$-ditosylat mit 0,41 g HOBt und 0,68 g DCC in 30 ml Dimethylformamid umgesetzt und gereinigt.
Ausb. 1,8 g.

160 mg der obigen Verbindung werden in 3 ml einer Mischung 90 %iger wäßriger Trifluoressigsäure/Dimercaptoethan wie 9:1 gelöst. Man läßt 1 Stunde bei Raumtemperatur stehen, engt ein, löst in Wasser und schüttelt 3 mal mit Diethylether aus. Die wäßrige Phase wird filtriert und gefriergetrocknet. Ausb. 140 mg, $[\alpha]_D^{21}$ = -25,2 ° (c = 1, in Wasser).

**Beispiel 5**

**[5-(5)-(3-Guanidino-propyl)-2,4-dioxo-imidazolidin-3-yl] acetyl-Asp-Val-OH**

Analog Beispiel 3c werden 438 mg [5-(R)-(3-Guanidino-propyl)-2,4-dioxo-imidazolidin-3-yl]essigsäure und 648 mg HCl•H-Asp(OtBu)-Val-OtBu mit 230 mg HOBt und 374 mg DCC in 20 ml Dimethylformamid umgesetzt und gereinigt. Ausb. 892 mg.

Obige Substanz wird analog Beispiel 3d in 9 ml 90 %iger Trifluoressigsäure umgesetzt.
Ausb. 767 mg, $[\alpha]_D^{21}$ = -36,6 ° (c = 1, in Wasser).

**Beispiel 6**

**[5-(S)-(3-Guanidino-propyl)-2,4-dioxo-imidazolidin-3-yl] acetyl-Asp-Val-$NH_2$**

Analog Beispiel 3c werden 772 g [5-(S)-(3-Guanidino-propyl)--2,4-dioxo-imidazolidin-3-yl]essigsäure und 971 mg HCl•H-Asp-(OtBu)-Val-$NH_2$ mit 410 mg HOBt und 680 mg DCC in 30 ml Dimethylformamid umgesetzt und gereinigt.
Ausb. 950 mg, $[\alpha]_D^{21}$ = -41,2 ° (c = 1, in Wasser).

Oben gewonnene Substanz wird analog Beispiel 3d in 10 ml 90 %iger Trifluoressigsäure umgesetzt. Ausb. 780 mg, $[\alpha]_D^{21}$ = -43,1° (c = 1, in Wasser).

**Beispiel 7**

**[5-(S)-(3-Guanidino-propyl)-2,4-dioxo-imidazolidin-3-yl] acetyl-Asp-Arg-Val-$NH_2$-acetat**

Analog Beispiel 3c werden 515 mg [5-(R)-(3-Guanidino-propyl)-2,4-dioxo-imidazolidin-3-yl]essigsäure und 1,58 g H-Asp(OtBu)-Arg-Val-$NH_2$-ditosylat mit 270 mg HOBt und 440 mg DCC in 20 ml Dimethylformamid umgesetzt und gereinigt.
Ausb. 950 mg.

430 mg der oben gewonnenen Substanz werden analog Beispiel 3d in 5 ml 90 %iger Trifluoressigsäure umgesetzt.
Ausb. 380 mg, $[\alpha]_D^{20}$ = -23,2° (c = 1, in Wasser)

**Beispiel 8**

**[5-(S)-(3-Guanidino-propyl)-2,4-dioxo-imidazolidin-3-yl] acetyl-Asp-Arg-isobutylamid-acetat**

Analog Beispiel 3c werden 515 mg [5-(S)-(3-Guanidino-propyl)-2,4-dioxo-imidazolidin-3-yl]essigsäure und 1,49 g H-Asp(OtBu)-Arg-isobutylamid-ditosylat mit 270 mg HOBt und 440 mg DCC in 20 ml Dimethylformamid umgesetzt und gereinigt. Ausb. 1,2 g.

360 mg der oben gewonnenen Substanz werden analog Beispiel 3d in 5 ml 90 %iger Trifluoressigsäure umgesetzt.
Ausb. 370 mg, $[\alpha]_D^{20}$ = -28,3° (c = 1, in Wasser).

**Beispiel 9**

**[5-(S)-(3-Guanidino-propyl)-2,4-dioxo-imidazolidin-3-yl] acetyl-Asp-Gln-OH**

Analog Beispiel 3c werden 770 mg [5-(S)-(3-Guanidino-propyl)-2,4-dioxo-imidazolidin-3-yl]essigsäure und 1,23 g HCl•H-Asp(OtBu)-Gln-OtBu mit 410 mg HOBt und 660 mg DCC in 20 ml Dimethylformamid umgesetzt und gereinigt.
Ausb. 891 mg.

850 mg oben gewonnener Substanz werden analog Beispiel 3d in 8 ml 90 %iger Trifluoressigsäure umgesetzt.
Ausb. 742 mg, $[\alpha]_D^{21}$ = -29,5° (c = 1, in Wasser)

**Beispiel 10**

**[5-(S)-(3-Guanidino-propyl)-2,4-dioxo-imidazolidin-3-yl] acetyl-Asp-Arg-4-Abu-OH-acetat**

Analog Beispiel 3c werden 515 mg [5-(S)-(3-Guanidino-propyl)-2,4-dioxo-imidazolidin-3-yl]essigsäure und 1,12 g 2 HCl•H-Asp(OtBu)-Arg-4-Abu-OtBu mit 270 mg HOBt und 440 mg DCC in 20 ml Dimethylformamid umgesetzt und gereinigt.
Ausb. 550 mg.

300 mg der oben gewonnenen Substanz werden analog Beispiel 3d in 3 ml 90 %iger Trifluoressigsäure umgesetzt.
Ausb. 288 mg, $[\alpha]_D^{21}$ = -29,8° (c = 1, in Wasser).

**Beispiel 11**

**[5-(S)-(3-Guanidino-propyl)-2,4-dioxo-imidazolidin-3-yl] acetyl-Asp-Ser-OH**

Analog Beispiel 3c werden 770 mg [5-(S)-(3-Guanidino-propyl)-2,4-dioxo-imidazolidin-3-yl]essigsäure und 1,27 g HCl•H-Asp(OtBu)-Ser(tBu)-OtBu mit 410 mg HOBt und 660 mg DCC in 20 ml Dimethylformamid umgesetzt und an Kieselgel (Laufmittel: Methylenchlorid/Methanol/Eisessig/Wasser wie 8:2:0,2:0,2) chromatographiert.

700 mg der oben gewonnenen Substanz werden analog Beispiel 3d in 7 ml 90 %iger Trifluoressigsäure umgesetzt.
Ausb. 579 mg, $[\alpha]_D^{21}$ = -19,1° (c = 1, in Wasser).

**Beispiel 12**

**[5-(S)-(3-Guanidino-propyl)-2,4-dioxo-imidazolidin-3-yl] acetyl-Asp-Arg-Hyp-NH-$C_2H_5$-acetat**

Analog Beispiel 3c werden 515 mg [5-(S)-(3-Guanidino-propyl)-2,4-dioxo-imidazolidin-3-yl]essigsäure und 1,66 g H-Asp(OtBu)-Arg-Hyp-$NHC_2H_5$-ditosylat mit 270 mg HOBt und 440 mg DCC in 20 ml Dimethylformamid umgesetzt und gereinigt.
Ausb. 947 mg

600 mg der oben gewonnenen Substanz werden analog Beispiel 3d in 6 ml 90 %iger Trifluoressigsäure umgesetzt.
Ausb. 574 mg, $[\alpha]_D^{21}$ = -43,7° (c = 1, in Wasser).

**Beispiel 13**

**[5-(S)-(3-Guanidino-propyl)-2,4-dioxo-imidazolidin-3-yl] acetyl-Asp-Phe-Azagly-$NH_2$**

Analog Beispiel 3c werden 600 mg [5-(S)-(3-Guanidino-propyl)-2,4-dioxo-imidazolidin-3-yl]essigsäure und 1 g HCl•H-Asp(OtBu)-Phe-Azagly-$NH_2$ mit 327 mg HOBt und 490 mg DCC in 15 ml Dimethylformamid umgesetzt und gereinigt.
Ausb. 800 mg.

540 mg der oben gewonnenen Substanz werden analog Beispiel 3d in 10 ml 90 %iger Trifluoressigsäure umgesetzt.
Ausb. 500 mg, $[\alpha]_D^{21}$ = -35° (c = 1, in Eisessig).

**Beispiel 14**

**[5-(S)-(3-Guanidino-propyl)-2,4-dioxo-imidazolidin-3-yl] acetyl-Asp-Phe-N($CH_3$)-NH-CO-$NH_2$**

Analog Beispiel 3c werden 257 mg [5-(S)-(3-Guanidino-propyl)-2,4-dioxo-imidazolidin-3-yl]essigsäure und 540 mg HCl•H-Acp(OtBu)-Phe-N($CH_3$)-NH-CO-$NH_2$ mit 185 mg HOBt und 278 mg DCC in 10 ml Dimethylformamid umgesetzt und auf Kieselgel (Eisessig/n-Butanol/Wasser wie 1:8:1) gereinigt.
Ausb. 670 mg.

460 mg der oben gewonnenen Substanz werden analog Beispiel 3d in 20 ml 90 %iger Trifluoressigsäure umgesetzt.
Ausb. 400 mg, $[\alpha]_D^{20}$ = -10° (c = 1, in Eisessig).

**Beispiel 15**

**[5-(S)-(3-Guanidino-propyl)-2,4-dioxo-imidazolidin-3-yl] acetyl-Asp-Phe-N(2-naphthylmethyl)-NH-CO-$NH_2$**

Analog Beispiel 3c werden 169 mg [5-(S)-(3-Guanidino-propyl)-2,4-dioxo-imidazolidin-3-yl]essigsäure und 380 mg HCl•H-Asp(OtBu)-Phe-N(2-naphthylmethyl)-NH-CO-$NH_2$ mit 93 mg HOBt und 139 mg DCC in 10 ml Dimethylformamid umgesetzt und an Kieselgel (Methylenchlorid/Methanol/Wasser/Essigsäure wie 8:2:0,2:0,2) reinigt.
Ausb. 100 mg
Oben gewonnene Substanz wird analog Beispiel 3d in 10 ml 90%iger Trifluoressigsäure umgesetzt.
Ausb. 90 mg, $[\alpha]_D^{23}$ = -11,2° (c = 1, in Eisessig).

**Beispiel 16**

**[5-(S)-(3-Guanidino-propyl)-2,4-dioxo-imidazolidin-3-yl] acetyl-Asp-Phe-N($C_2H_5$)-NH-CO-$NH_2$**

Analog Beispiel 3c werden 365 mg [5-(R)-(3-Guanidino-propyl)-2,4-dioxo-imidazolidin-3-yl]essigsäure und 600 mg HCl•H-Asp(OtBu)-Phe-N($C_2H_5$)-NH-CO-$NH_2$ mit 199 mg HOBt und 298 mg DCC in 5 ml Dimethylformamid umgesetzt und auf Kieselgel (n-Butanol/Wasser/Eisessig wie 8:1:1) gereinigt.
Ausb. 400 mg
390 mg der oben gewonnenen Substanz werden analog Beispiel 3d in 15 ml 90 %iger Trifluoressigsäure umgesetzt.
Ausb. 380 mg, $[\alpha]_D^{21}$ = -12,7° (c = 1,in Eisessig).

**Beispiel 17**

**[5-(S)-(3-Guanidino-propyl)-2,4-dioxo-imidazolidin-3-yl] acetyl-Asp-Trp-Pro-OH**

Analog Beispiel 3c werden 515 mg [5-(S)-(3-Guanidino-propyl)-2,4-dioxo-imidazolidin-3-yl]essigsäure und 1,154 g HCl•H-Asp(OtBu)-Trp-Pro-OtBu mit 270 mg HOBt und 440 mg DCC in 20 ml Dimethylformamid umgesetzt und an Kieselgel (Methylenchlorid/Methanol/Eisessig/Wasser wie 8:2:0,15:0,15) gereinigt.
Ausb. 850 mg.

790 mg oben gewonnener Substanz werden analog Beispiel 4 in 10 ml 90 %iger Trifluoressigsäure/1,2-Ethandithiol (9:1) umgesetzt.
Ausb. 750 mg, $[\alpha]_D^{22}$ = -45,5° (c = 1, in Wasser).

**Beispiel 18**

**[5-(S)-(3-Guanidino-propyl)-2,4-dioxo-imidazolidin-3-yl] acetyl-Asp-Trp-OH**

Analog Beispiel 3c werden 770 mg [5-(S)-(3-Guanidino-propyl)-2,4-dioxo-imidazolidin-3-yl]essigsäure und 1,4 g HCl•H-Asp(OtBu)-Trp-OtBu mit 410 mg HOBt und 660 mg DCC umgesetzt und an Kieselgel (Methylenchlorid/Methanol/Eisessig/Wasser wie 8:2:0,2:0,2) gereinigt.
Ausb. 760 mg.

700 mg oben gewonnener Substanz werden analog Beispiel 4 in 11 ml 90 %iger Trifluoressigsäure/1,2-Ethandithiol (9:1) umgesetzt.
Ausb. 617 mg, $[\alpha]_D^{21}$ = -12,3° (c = 1, in Wasser)

**Beispiel 19**

**[5-(S)-(3-Guanidino-propyl)-2,4-dioxo-imidazolidin-3-yl] acetyl-Asp-Trp-Gly-OH**

Analog Beispiel 3c werden 1,21 g [5-(S)-(3-Guanidino-propyl)-2,4-dioxo-imidazolidin-3-yl]essigsäure und 2,47 g HCl•H-Asp(OtBu)-Trp-Gly-OtBu mit 635 mg HOBt und 635 mg DCC in 30 ml Dimethylformamid umgesetzt und an Kieselgel (Methylenchlorid/Methanol/Eisessig/Wasser wie 9:2:0,2:0,2) gereinigt.
Ausb. 1 g Öl.

Oben gewonnene Substanz wird analog Beispiel 4 in 10 ml 90 %iger Trifluoressigsäure/1,2-Ethandithiol (9:1) umgesetzt.
Ausb. 0,67 g, $[\alpha]_D^{23}$ = -28,2° (c = 1, in Wasser).

**Beispiel 20**

**[5-(S)-(3-Guanidino-propyl)-2,4-dioxo-imidazolidin-3-yl] acetyl-Asp-Trp-$NH_2$**

Aus [5-(S)-(3-Guanidino-propyl)-2,4-dioxo-imidazolidin-3-yl]essigsäure und HCl•H-Asp(OtBu)-Trp-$NH_2$ analog Beispiel 3c und Beispiel 4.
$[\alpha]_D^{24}$ = -19° (c = 1, in Wasser)

**Beispiel 21**

**[5-(S)-(3-Guanidino-propyl)-2,4-dioxo-imidazolidin-3-yl] acetyl-Asp-NH-$(CH_2)_4$-$NH_2$-acetat**

Aus [5-(S)-(3-Guanidino-propyl)-2,4-dioxo-imidazolidin-3-yl]essigsäure und HCl•H-Asp(OtBu)-NH-$(CH_2)_6$-NH-Boc analog Beispiel 3c und Beispiel 3d.
$[\alpha]_D^{21}$ = -22,2° (c = 1, in Wasser)

**Beispiel 22**

**[5-(S)-(3-Guanidino-propyl)-2,4-dioxo-imidazolidin-3-yl] acetyl-Asp-L-phenylglycin**

Aus [5-(S)-(3-Guanidino-propyl)-2,4-dioxo-imidazolidin-3-yl]essigsäure und HCl•H-Asp(OtBu)-Phg-OtBu analog Beispiel 3c und Beispiel 3d.
$[\alpha]_D^{19}$ = +20,7° (c = 1, in Wasser)

**Beispiel 23**

**[5-(S)-(3-Guanidino-propyl)-2,4-dioxo-imidazolidin-3-yl] acetyl-Asp-L-hexahydrophenylglycin**

Aus [5-(S)-(3-Guanidino-propyl)-2,4-dioxo-imidazolidin-3-yl]essigsäure und HCl•H-Asp(OtBu)-L-hexahydrophenylglycin-OtBu analog Beispiel 3c und Beispiel 3d.
$[\alpha]_D^{20}$ = -30,6° (c = 1, in Wasser)

**Beispiel 24**

**[5-(S)-(3-Guanidino-propyl)-2,4-dioxo-imidazolidin-3-yl] acetyl-Asp-L-α-Phenylglycinol**

Aus [5-(S)-(3-Guanidino-propyl)-2,4-dioxo-imidazolidin-3-yl]essigsäure und HCl•H-Asp(OtBu)-L-α-phenylglycinol analog Beispiel 3c und Beispiel 3d.
$[\alpha]_D^{20}$ = -9,8° (c = 1, in Wasser)

**Beispiel 25**

**[5-(S)-(3-Guanidino-propyl)-2,4-dioxo-imidazolidin-3-yl] acetyl-Asp-D-α-Phenylglycinol**

Aus [5-(S)-(3-Guanidino-propyl)-2,4-dioxo-imidazolidin-3-yl]essigsäure und HCl•H-Asp(OtBu)-D-α-phenylglycinol analog Beispiel 3c und Beispiel 3d.
$[\alpha]_D^{19}$ = -49,8° (c = 1, in Wasser)

**Beispiel 26**

**[5-(S)-(3-Guanidino-propyl)-2,4-dioxo-imidazolidin-3-yl] acetyl-Asp-Tyr-OH**

Aus [5-(S)-(3-Guanidino-propyl)-2,4-dioxo-imidazolidin-3-yl]essigsäure und HCl•H-Asp(OtBu)-Tyr(tBu)-OtBu analog Beispiel 3c und Beispiel 4.
$[\alpha]_D^{21}$ = -17° (c = 1, in Wasser)

**Beispiel 27**

**[5-(S)-(3-Guanidino-propyl)-2,4-dioxo-imidazolidin-3-yl] acetyl-Asp-Tyr-$NH_2$**

Aus [5-(S)-(3-Guanidino-propyl)-2,4-dioxo-imidazolidin-3-yl]essigsäure und HCl•H-Asp(OtBu)-Tyr(tBu)-$NH_2$ analog Beispiel 3c und Beispiel 4.
$[\alpha]_D^{21}$ = -22,5° (c = 1, in Wasser)

**Beispiel 28**

**[5-(S)-(3-Guanidino-propyl)-2,4-dioxo-imidazolidin-3-yl] acetyl-Asp-Nal-OH**

Aus [5-(S)-(3-Guanidino-propyl)-2,4-dioxo-imidazolidin-3-yl]essigsäure und HCl•H-Asp(OtBu)-Nal-OtBu analog Beispiel 3c und Beispiel 4.
$[\alpha]_D^{21}$ = -8,7° (c = 1, in Wasser)

**Beispiel 29**

**[5-(S)-(3-Guanidino-propyl)-2,4-dioxo-imidazolidin-3-yl] acetyl-Asp-Nal-Aoc-OH**

Aus [5-(S)-(3-Guanidino-propyl)-2,4-dioxo-imidazolidin-3-yl]essigsäure und HCl•H-Asp(OtBu)-Nal-Aoc-OtBu analog Beispiel 3c und Beispiel 4.
$[\alpha]_D^{23}$ = -15,3° (c = 1, in Wasser)

**Beispiel 30**

**[5-(S)-(3-Guanidino-propyl)-2,4-dioxo-imidazolidin-3-yl] acetyl-Asp-(fluoren-9-yl)-amid**

**a) [5-(S)-(3-Nitro-guanidino-propyl)-2,4-dioxo-imidazolidin-3-yl]essigsäure**

11 g (0,05 mol) H-Arg($NO_2$)-OH werden mit 4,3 g (0,05 mol) Natriumhydrogencarbonat in 200 ml Wasser unter Rückfluß gelöst. Bei 70° werden 7,1 g (0,055 mol) Isocyanatoessigsäureethylester zugetropft. Man rührt 1 Stunde bei 70°C, läßt abkühlen, rührt über Nacht bei Raumtemperatur, versetzt mit 50 ml konz. Salzsäure, engt ein, gibt 20 ml halbkonz. Salzsäure zu, erhitzt 1 Stunde unter Rückfluß, engt weitgehend ein und saugt das ausgefallene Produkt ab.
Schmp. 202-207°C.

**b) [5-(S)-(3-Nitro-guanidino-propyl)-2,4-dioxo-imidazolidin-3-yl]acetyl-Asp(OBzl)-fluoren-9-yl)-amid**

0,15 g (0,5 mmol) [5-(S)-(3-Nitro-guanidino-propyl)-2,4-dioxo-imidazolidin-3-yl]essigsäure werden in 5 ml Dimethylformamid gelöst. Nach Zugabe von 0,13 g (0,5 mmol) Disuccinimidylcarbonat und 0,05 g 4-Dimethylaminopyridin wird 1,5 Stunden bei Raumtemperatur gerührt, dann werden 0,2 ml (2,5 mmol) N-Ethylmorpholin und 0,47 g (0,5 mmol) H-Asp(OBzl)-(fluoren-9-yl)-amid-trifluoracetat in 3 ml Dimethylformamid zugegeben und über Nacht bei Raumtemperatur gerührt. Man engt zur Trockene ein, versetzt mit Methylenchlorid und extrahiert mit Natriumhydrogencarbonat- und Kaliumhydrogensulfatlösung. Die organische Phase wird eingeengt und der Rückstand mit Methanol/Essigester kristallisiert.
Ausb. 150 mg, Schmp. 162-164°C

**c) [5-(S)-(3-Guanidino-propyl)-2,4-dioxo-imidazolidin-3-yl] acetyl-Asp-(fluoren-9-yl)-amid**

80 mg [5-(S)-(3-Nitro-guanidino-propyl)-2,4-dioxo-imidazolidin-3-yl]acetyl-Asp(OBzl)-(fluoren-9-yl)-amid werden in 50 ml Dimethylformamid gelöst und nach Zugabe von 0,1 g 10 %-Pd/C 8 Stunden bei 50°C hydriert. Dann werden 50 ml Wasser zugegeben und weitere 8 Stunden bei 50°C hydriert. Man filtriert heiß ab, engt ein, verrührt mit Isopropanol und kristallisiert aus Methanol um.
Ausb. 58 mg, Schmp. > 250°C.

**Patentansprüche**

**Patentansprüche für folgende Vertragsstaaten : BE, DE, DK, FR, GB, IT, LU, NL**

**1.** Verbindung der allgemeinen Formel I

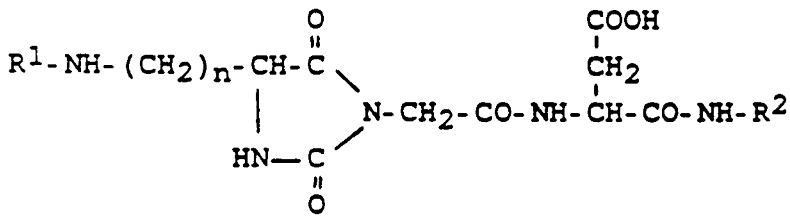

(I)

in welcher

| | |
|---|---|
| n | für eine ganze Zahl 3 oder 4 steht; |
| $R^1$ | $C_1$-$C_6$-Alkyl oder einen Rest der Formel II bedeutet |

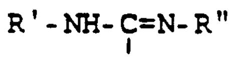

(II)

worin

| | |
|---|---|
| R' und R" | unabhängig voneinander Wasserstoff oder $C_1$-$C_6$-Alkyl bedeuten; |
| $R^2$ | Wasserstoff, -NH-CO-$NH_2$ oder $C_1$-$C_{18}$-Alkyl bedeutet, das gegebenenfalls ein- oder mehrfach durch gleiche oder verschiedene Reste aus der Reihe Hydroxy, Carbamoyl, Carboxy, Carboxamido, Amino, Mercapto, $C_1$-$C_{18}$-Alkoxy, Guanidino, $C_3$-$C_8$-Cycloalkyl, Halogen, Nitro, Trifluormethyl und einen Rest $R^3$ substituiert ist, wobei |
| $R^3$ | für $C_6$-$C_{14}$-Aryl, $C_6$-$C_{14}$-Aryl-$C_1$-$C_8$-alkyl oder einen mono- oder bizyklischen 5- bis 12-gliedrigen heterozyklischen Ring, der aromatisch, teilhydriert oder vollständig hydriert sein kann und der als Heteroelement ein, zwei oder drei gleiche oder verschiedene Stickstoff-, Sauerstoff- oder Schwefel-Atome enthalten kann, steht, wobei der Aryl- und unabhängig voneinander der Heterozyklus-Rest gegebenenfalls ein- oder mehrfach durch gleiche oder verschiedene Reste aus der Reihe $C_1$-$C_{18}$-Alkyl, $C_1$-$C_{18}$-Alkoxy, Halogen, Hydroxy Nitro und Trifluormethyl substituiert sind; oder für einen Rest $R^4$ steht; wobei |
| $R^4$ | $NR^5R^6$; $OR^5$; $SR^5$; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest bedeutet, bei dem die Peptidbindung zu NH-$CH_2$ reduziert sein kann, sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen Rest $COR^{4'}$, bedeutet worin $R^{4'}$ wie $R^4$ definiert ist; |
| $R^5$ | Wasserstoff, gegebenenfalls durch eine Aminogruppe substituiertes $C_1$-$C_{18}$-Alkyl, $C_6$-$C_{14}$-Aryl, $C_6$-$C_{14}$-Aryl-$C_1$-$C_8$-alkyl, $C_1$-$C_{18}$-Alkylcarbonyl, $C_1$-$C_{18}$-Alkyloxycarbonyl, $C_6$-$C_{14}$-Arylcarbonyl, $C_6$-$C_{12}$-Aryl-$C_1$-$C_8$-alkylcarbonyl, $C_6$-$C_{18}$-Aryl-$C_1$-$C_{18}$-alkyloxycarbonyl, einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest bedeutet, bei dem die Peptidbindung zu NH-$CH_2$ reduziert sein kann; |
| $R^6$ | Wasserstoff, $C_1$-$C_{18}$-Alkyl, $C_6$-$C_{12}$-Aryl oder $C_6$-$C_{12}$-Aryl-$C_1$-$C_8$-alkyl bedeutet; |

sowie deren physiologisch verträgliche Salze.

**2.** Verbindung der Formel I gemäß Anspruch 1, dadurch gekennzeichnet, daß

| | |
|---|---|
| $R^2$ | Wasserstoff oder $C_1$-$C_8$-Alkyl bedeutet, das gegebenenfalls 1- bis 4-fach durch gleiche oder verschiedene Reste aus der Reihe Hydroxy, Amino, Carboxy, Carbamoyl, Guanidino, $C_1$-$C_6$-Alkoxy, $C_3$-$C_6$-Cycloalkyl, Halogen und einen Rest $R^3$ substi- |

tuiert ist, wobei

$R^3$ für $C_6$-$C_{12}$-Aryl, $C_6$-$C_{12}$-Aryl-$C_1$-$C_4$-alkyl oder einen mono- oder bizyklischen 5- bis 12-gliedrigen heterozyklischen, aromatischen Ring, steht, der als Heteroelement ein oder zwei Stickstoffatome enthält, wobei unabhängig voneinander der Aryl- und Heterozyklus-Rest gegebenenfalls ein-, zwei- oder dreifach durch gleiche oder verschiedene Reste aus der Reihe $C_1$-$C_6$-Alkyl, $C_1$-$C_6$-Alkoxy, Halogen, Nitro, Hydroxy und Trifluormethyl substituiert sind; oder für einen Rest $R^4$ steht, wobei

$R^4$ $NR^5R^6$; $OR^5$; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure-oder einen Dipeptid-Rest sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen Rest $COR^{4'}$ bedeutet, worin $R^{4'}$ wie $R^4$ definiert ist;

$R^5$ und $R^6$ unabhängig voneinander Wasserstoff, $C_1$-$C_8$-Alkyl oder $C_6$-$C_{12}$-Aryl bedeutet.

**3.** Verbindung der Formel I gemäß den Ansprüchen 1 bis 2, dadurch gekennzeichnet, daß

$R^1$ ein Rest der Formel II bedeutet, worin

R' und R'' unabhängig voneinander Wasserstoff oder $C_1$-$C_2$-Alkyl bedeuten;

$R^2$ Wasserstoff oder $C_1$-$C_6$-Alkyl bedeutet, das zweifach durch gleiche oder verschiedene Reste $R^4$ substituiert ist, wobei

$R^4$ Hydroxy; Amino; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest bedeutet, bei dem die Peptidbindung zu $NH$-$CH_2$ reduziert sein kann, sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen Rest $COR^{4'}$ bedeutet, worin $R^{4'}$ wie $R^4$ definiert ist.

**4.** Verbindung der Formel I gemäß den Ansprüchen 1 bis 3, dadurch gekennzeichnet, daß

$R^2$ Methyl bedeutet, das zweifach durch verschiedene Reste $R^4$ und $COR^{4'}$ substituiert ist, wobei $R^4$ bzw. $R^{4'}$ wie in Anspruch 3 definiert sind.

**5.** Verbindung der Formel I, gemß den Ansprüchen 1 bis 4, dadurch gekennzeichnet, daß

$R^1$ ein Rest der Formel II bedeutet, worin

R' und R'' Wasserstoff bedeuten

$R^2$ Wasserstoff oder $C_1$-$C_6$-Alkyl bedeutet, das ein- oder zweifach, unabhängig voneinander durch -OH, $NH_2$, -COOH, -$CONH_2$, -NH-C($NH_2$)=NH , $C_5$-$C_8$-Cycloalkyl, einen Rest $R^3$, wobei

$R^3$ für $C_6$-$C_{14}$-Aryl, das gegebenenfalls durch Hydroxy substituiert sein kann, einen bizyklischen 8- bis 12-gliedrigen heterozyklischen, aromatischen Ring, der als Heteroelement ein Stickstoffatom enthält, steht,
oder einen Rest $R^4$ substituiert ist, wobei

$R^4$ für $NR^5R^6$, einen natürlichen oder unnatürlichen Aminosäure-, Iminosäure-, gegebenenfalls N-$C_1$-$C_4$-alkylierten oder N-$C_6$-$C_{14}$-aryl-$C_1$-$C_4$-alkylierten Azaaminosäure- oder Dipeptid-Rest sowie deren Amide, oder einen Rest $COR^{4'}$, worin $R^{4'}$ wie $R^4$ definiert ist, steht,

$R^5$ Wasserstoff,

$R^6$ $C_1$-$C_6$-Alkyl bedeuten, und

n 3 bedeutet.

**6.** Verfahren zur Herstellung einer Verbindung der Formel I gemäß den Ansprüchen 1 bis 4, dadurch gekennzeichnet, daß man

a1) Verbindungen der Formel IV

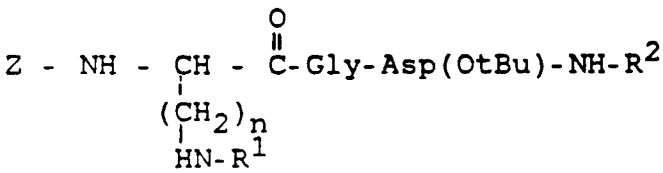 (IV),

in der Z, $R^1$, $R^2$ und n die in den Ansprüchen 1 bis 5 angegebenen Bedeutungen haben, in Tetrahydrofuran 2-3 Stunden im Rückfluß erhitzt,

oder

a2) die Verbindungen der allgemeinen Formel VI und VII

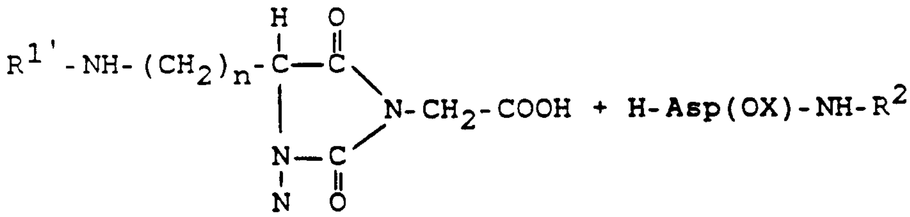

(VI) (VII)

worin

$R^{1'}$ $-C(NH_2)=N-NO_2$ bedeutet oder wie $R^1$ oben definiert ist,

X für tBu oder Bzl steht und $R^2$ und n die oben beschriebenen Bedeutungen haben,

mittels der allgemeinen Methoden der Peptidchemie kondensiert, und

b) die so entstandenen Hydantoine der allgemeinen Formel V

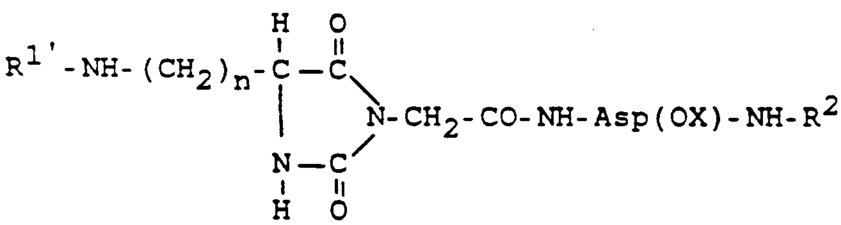 (V)

durch katalytische Hydrierung und/oder saure Abspaltung der Schutzgruppen in die erfindungsgemäßen Verbindungen der allgemeinen Formel I überführt.

7. Verbindung der Formel I gemäß den Ansprüchen 1 bis 5 zur Anwendung als Heilmittel.

8. Verbindung der Formel I gemäß den Ansprüchen 1 bis 5 zur Hemmung der Thrombozytenaggregation.

9. Verbindung der Formel I gemäß den Ansprüchen 1 bis 5 zur Hemmung der Osteoclastenbindung an die Knochenoberfläche.

10. Pharmazeutische Zubereitung enthaltend eine Verbindung der Formel I gemäß den Ansprüchen 1 bis 5 oder deren physiologisch verträgliches Salz und einen physiologisch annehmbaren Träger.

**Patentansprüche für folgende Vertragsstaaten: ES, GR**

1. Verfahren zur Herstellung einer Verbindung der allgemeinen Formel I

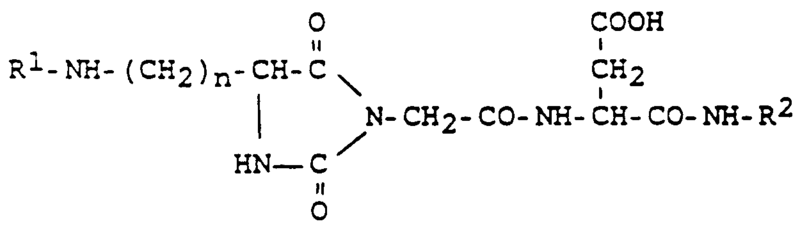

(I)

in welcher

n für eine ganze Zahl 3 oder 4 steht;

$R^1$ $C_1$-$C_6$-Alkyl oder einen Rest der Formel II bedeutet

$$R'-NH-\underset{|}{C}=N-R'' \qquad (II)$$

worin

R' und R'' unabhängig voneinander Wasserstoff oder $C_1$-$C_6$-Alkyl bedeuten;

$R^2$ Wasserstoff, -NH-CO-$NH_2$ oder $C_1$-$C_{18}$-Alkyl bedeutet, das gegebenenfalls ein- oder mehrfach durch gleiche oder verschiedene Reste aus der Reihe Hydroxy, Carbamoyl, Carboxy, Carboxamido, Amino, Mercapto, $C_1$-$C_{18}$-Alkoxy, Guanidino, $C_3$-$C_8$-Cycloalkyl, Halogen, Nitro, Trifluormethyl und einen Rest $R^3$ substituiert ist, wobei

$R^3$ für $C_6$-$C_{14}$-Aryl, $C_6$-$C_{14}$-Aryl-$C_1$-$C_8$-alkyl oder einen mono- oder bizyklischen 5- bis 12-gliedrigen heterozyklischen Ring, der aromatisch, teilhydriert oder vollständig hydriert sein kann und der als Heteroelement ein, zwei oder drei gleiche oder verschiedene Stickstoff-, Sauerstoff- oder Schwefel-Atome enthalten kann, steht, wobei der Aryl- und unabhängig voneinander der Heterozyklus-Rest gegebenenfalls ein- oder mehrfach durch gleiche oder verschiedene Reste aus der Reihe $C_1$-$C_{18}$-Alkyl, $C_1$-$C_{18}$-Alkoxy, Halogen, Hydroxy, Nitro und Trifluormethyl substituiert sind; oder für einen Rest $R^4$ steht;
wobei

$R^4$ $NR^5R^6$; $OR^5$; $SR^5$; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest bedeutet, bei dem die Peptidbindung zu NH-$CH_2$ reduziert sein kann, sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen Rest $COR^{4'}$, bedeutet worin $R^{4'}$ wie $R^4$ definiert ist;

$R^5$ Wasserstoff, gegebenenfalls durch eine Aminogruppe substituiertes $C_1$-$C_{18}$-Alkyl, $C_6$-$C_{14}$-Aryl, $C_6$-$C_{14}$-Aryl-$C_1$-$C_8$-alkyl, $C_1$-$C_{18}$-Alkylcarbonyl, $C_1$-$C_{18}$-Alkyloxycarbonyl, $C_6$-$C_{14}$-Arylcarbonyl, $C_6$-$C_{12}$-Aryl-$C_1$-$C_8$-alkylcarbonyl, $C_1$-$C_{18}$-Aryl-$C_1$-$C_{18}$-alkyloxycarbonyl, einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipebtid-Rest bedeutet, bei dem die Peptidbindung zu NH-$CH_2$ reduziert sein kann;

$R^6$ Wasserstoff, $C_1$-$C_{18}$-Alkyl, $C_6$-$C_{12}$-Aryl oder $C_6$-$C_{12}$-Aryl-$C_1$-$C_8$-alkyl bedeutet;

sowie deren physiologisch verträgliche Salze, dadurch gekennzeichnet, daß man Verbindungen der Formel IV

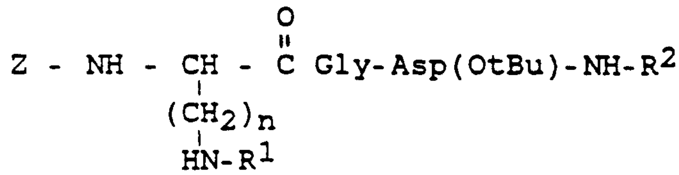 (IV),

in der $R^1$, $R^2$ und n die in den Ansprüchen 1 bis 4 angegebenen Bedeutungen haben, in Trifluoressigsäure löst und 30 Minuten - 3 Stunden bei Raumtemperatur stehen läßt.

**2.** Verfahren gemäß Anspruch 1, dadurch gekennzeichnet, daß man eine Verbindung der Formel I herstellt, worin

| | |
|---|---|
| $R^2$ | Wasserstoff oder $C_1$-$C_8$-Alkyl bedeutet, das gegebenenfalls 1- bis 4-fach durch gleiche oder verschiedene Reste aus der Reihe Hydroxy, Amino, Carboxy, Carbamoyl, Guanidino, $C_1$-$C_6$-Alkoxy, $C_3$-$C_6$-Cycloalkyl, Halogen und einen Rest $R^3$ substituiert ist, wobei |
| $R^3$ | für $C_6$-$C_{12}$-Aryl, $C_6$-$C_{12}$-Aryl-$C_1$-$C_4$-alkyl oder einen mono- oder bizyklischen 5- bis 12-gliedrigen heterozyklischen, aromatischen Ring, steht, der als Heteroelement ein oder zwei Stickstoffatome enthält, wobei unabhängig voneinander der Aryl- und Heterozyklus-Rest gegebenenfalls ein-, zwei- oder dreifach durch gleiche oder verschiedene Reste aus der Reihe $C_1$-$C_6$-Alkyl, $C_1$-$C_6$-Alkoxy, Halogen, Nitro, Hydroxy und Trifluormethyl substituiert sind; oder für einen Rest $R^4$ steht, wobei |
| $R^4$ | $NR^5R^6$; $OR^5$; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure-oder einen Dipeptid-Rest sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen Rest $COR^{4'}$ bedeutet, worin $R^{4'}$ wie $R^4$ definiert ist; |
| $R^5$ und $R^6$ | unabhängig voneinander Wasserstoff, $C_1$-$C_8$-Alkyl oder $C_6$-$C_{12}$-Aryl bedeutet. |

**3.** Verfahren gemäß den Ansprüchen 1 bis 2, dadurch gekennzeichnet, daß man eine Verbindung der Formel I herstellt, worin

| | |
|---|---|
| $R^1$ | ein Rest der Formel II bedeutet, worin |
| R' und R'' | unabhängig voneinander Wasserstoff oder $C_1$-$C_2$-Alkyl bedeuten; |
| $R^2$ | Wasserstoff oder $C_1$-$C_6$-Alkyl bedeutet, das zweifach durch gleiche oder verschiedene Reste $R^4$ substituiert ist, wobei |
| $R^4$ | Hydroxy; Amino; eine Aminosäureseitenkette; einen natürlichen oder unnatürlichen Aminosäure-, Iminosäure-, gegebenenfalls N-$C_1$-$C_8$-alkylierten oder $C_6$-$C_{14}$-Aryl-$C_1$-$C_8$-alkylierten Azaaminosäure- oder einen Dipeptid-Rest bedeutet, bei dem die Peptidbindung zu NH-$CH_2$ reduziert sein kann, sowie deren Ester und Amide, wobei freie funktionelle Gruppen gegebenenfalls durch Wasserstoff oder Hydroxymethyl substituiert oder durch in der Peptidchemie übliche Schutzgruppen geschützt sein können; oder einen $COR^{4'}$ bedeutet, worin $R^{4'}$ wie $R^4$ definiert ist. |

**4.** Verfahren gemäß den Ansprüchen 1 bis 3, dadurch gekennzeichnet, daß man eine Verbindung der Formel I herstellt, worin

$R^2$ Methyl bedeutet, das zweifach durch verschiedene Reste $R^4$ und $COR^{4'}$ substituiert ist, wobei $R^4$ bzw. $R^{4'}$ wie in Anspruch 3 definiert sind.

**5.** Verfahren gemäß den Ansprüchen 1 bis 4, dadurch gekennzeichnet, daß man eine Verbindung der Formel I herstellt, worin

| | |
|---|---|
| $R^1$ | ein Rest der Formel II bedeutet, worin |
| R' und R'' | Wasserstoff bedeuten |
| $R^2$ | Wasserstoff oder $C_1$-$C_6$-Alkyl bedeutet, das ein- oder zweifach, unabhängig voneinander durch -OH, $NH_2$, -COOH, -$CONH_2$, -NH-C($NH_2$)=NH , $C_5$-$C_8$-Cycloalkyl, einen Rest $R^3$, wobei |
| $R^3$ | für $C_6$-$C_{14}$-Aryl, das gegebenenfalls durch Hydroxy substituiert sein kann, einen |

bizyklischen 8- bis 12-gliedrigen heterozyklischen, aromatischen Ring, der als Heteroelement ein Stickstoffatom enthält, steht,
oder einen Rest $R^4$ substituiert ist, wobei

$R^4$ für $NR^5R^6$, einen natürlichen oder unnatürlichen Aminosäure-, Iminosäure-, gegebenenfalls $N-C_1-C_4$-alkylierten oder $N-C_6-C_{14}$-aryl-$C_1-C_4$-alkylierten Azaaminosäure- oder Dipeptid-Rest sowie deren Amide, oder einen Rest $COR^{4'}$, worin $R^{4'}$ wie $R^4$ definiert ist, steht,

$R^5$ Wasserstoff,

$R^6$ $C_1-C_6$-Alkyl bedeuten, und

n 3 bedeutet.

**6.** Verfahren zur Herstellung einer pharmazeutischen Zubereitung enthaltend eine Verbindung der Formel I gemäß den Ansprüchen 1 bis 5, dadurch gekennzeichnet, daß man diese und einen physiologisch annehmbaren Träger in eine geeignete Darreichungsform bringt.

**Claims**

**Claims for the following Contracting States : BE, DE, DK, FR, GB, IT, LU, NL**

**1.** A compound of the formula I

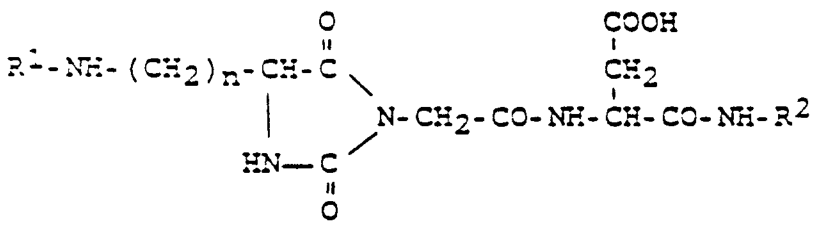

(I)

in which

n is an integer 3 or 4;

$R^1$ is $C_1-C_6$-alkyl or a radical of the formula II

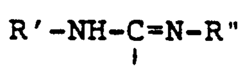

**(II)**

in which

R' and R" are, independently of one another, hydrogen or $C_1-C_6$-alkyl;

$R^2$ is hydrogen, $-NH-CO-NH_2$ or $C_1-C_{18}$-alkyl which is optionally substituted one or more times by identical or different radicals from the series comprising hydroxyl, carbamoyl, carboxyl, carboxamido, amino, mercapto, $C_1-C_{18}$-alkoxy, guanidino, $C_3-C_8$-cycloalkyl, halogen, nitro, trifluoromethyl and a radical $R^3$, where

$R^3$ is $C_6-C_{14}$-aryl, $C_6-C_{14}$-aryl-$C_1-C_8$-alkyl or a mono- or bicyclic 5- to 12-membered heterocyclic ring which can be aromatic, partially hydrogenated or completely hydrogenated and which can contain as hetero element one, two or three identical or different nitrogen, oxygen or sulfur atoms, where the aryl and, independently of one another, the heterocyclic radical is optionally substituted one or more times by identical or different radicals from the series comprising $C_1-C_{18}$-alkyl, $C_1-C_{18}$-alkoxy, halogen, hydroxyl, nitro and trifluoromethyl; or is a radical $R^4$; where

$R^4$ is $NR^5R^6$; $OR^5$; $SR^5$; an amino-acid side-chain; a residue of a natural or unnatural amino acid, imino acid, optionally $N-C_1-C_8$-alkylated or $C_6-C_{14}$-aryl-$C_1-C_8$-alkylated azaamino acid or a dipeptide in which the peptide linkage can be reduced to $NH-CH_2$, and the esters and amides thereof, where free functional groups can optionally be replaced by hydrogen or hydroxymethyl or protected by the protective groups customary in peptide chemistry; or is a radical $COR^{4'}$ in which $R^{4'}$ is defined as $R^4$;

$R^5$ is hydrogen, optionally amino group-substituted $C_1-C_{18}$-alkyl, $C_6-C_{14}$-aryl, $C_6-C_{14}$-

aryl-$C_1$-$C_8$-alkyl, $C_1$-$C_{18}$-alkylcarbonyl, $C_1$-$C_{18}$-alkyloxycarbonyl, $C_6$-$C_{14}$arylcarbonyl, $C_6$-$C_{12}$-aryl-$C_1$-$C_8$-alkylcarbonyl, $C_6$-$C_{18}$-aryl-$C_1$-$C_{18}$-alkyloxycarbonyl, a residue of a natural or unnatural amino acid, imino acid, optionally N-$C_1$-$C_8$-alkylated or $C_6$-$C_{14}$-aryl-$C_1$-$C_8$-alkylated azaamino acid or a dipeptide in which the peptide linkage can be reduced to NH-$CH_2$;

$R^6$ is hydrogen, $C_1$-$C_{18}$-alkyl, $C_6$-$C_{12}$-aryl or $C_6$-$C_{12}$-aryl-$C_1$-$C_8$-alkyl;

and the physiologically tolerated salts thereof.

**2.** A compound of the formula I as claimed in claim 1, wherein

$R^2$ is hydrogen or $C_1$-$C_8$-alkyl which is optionally substituted 1 to 4 times by identical or different radicals from the series comprising hydroxyl, amino, carboxyl, carbamoyl, guanidino, $C_1$-$C_6$-alkoxy, $C_3$-$C_6$-cycloalkyl, halogen and a radical $R^3$, where

$R^3$ is $C_6$-$C_{12}$-aryl, $C_6$-$C_{12}$-aryl-$C_1$-$C_4$-alkyl or a mono-or bicyclic 5- to 12-membered heterocyclic aromatic ring which contains as hetero element one or two nitrogen atoms, where, independently of one another, the aryl and heterocyclic radical are optionally substituted once, twice or three times by identical or different radicals from the series comprising $C_1$-$C_6$-alkyl, $C_1$-$C_6$-alkoxy, halogen, nitro, hydroxyl and trifluoromethyl; or is a radical $R^4$, where

$R^4$ is $NR^5R^6$; $OR^5$; an amino-acid side-chain; a residue of a natural or unnatural amino acid, imino acid, optionally N-$C_1$-$C_8$-alkylated or $C_6$-$C_{14}$-aryl-$C_1$-$C_8$-alkylated azaamino acid or a dipeptide, and the esters and amides thereof, where free functional groups can optionally be replaced by hydrogen or hydroxymethyl or protected by protective groups customary in peptide chemistry; or is a radical $COR^{4'}$ in which $R^{4'}$ is defined as $R^4$;

$R^5$ and $R^6$ are, independently of one another, hydrogen, $C_1$-$C_8$-alkyl or $C_6$-$C_{12}$-aryl.

**3.** A compound of the formula I as claimed in claims 1 and 2, wherein

$R^1$ is a radical of the formula II in which

R' and R'' are, independently of one another, hydrogen or $C_1$-$C_2$-alkyl;

$R^2$ is hydrogen or $C_1$-$C_6$-alkyl which is substituted twice by identical or different radicals $R^4$, where

$R^4$ is hydroxyl; amino; an amino-acid side-chain; a residue of a natural or unnatural amino acid, imino acid, optionally N-$C_1$-$C_8$-alkylated or $C_6$-$C_{14}$-aryl-$C_1$-$C_8$-alkylated azaamino acid or a dipeptide in which the peptide linkage can be reduced to NH-$CH_2$, and the esters and amides thereof, where free functional groups can optionally be replaced by hydrogen or hydroxymethyl or protected by protective groups customary in peptide chemistry; or is a radical $COR^{4'}$ in which $R^{4'}$ is defined as $R^4$.

**4.** A compound of the formula I as claimed in claims 1 to 3, wherein

$R^2$ is methyl which is substituted twice by different radicals $R^4$ and $COR^{4'}$ where $R^4$ and $R^{4'}$ are defined as in claim 3.

**5.** A compound of the formula I as claimed in claims 1 to 4, wherein

$R^1$ is a radical of the formula II in which R' and R'' are hydrogen,

$R^2$ is hydrogen or $C_1$-$C_6$-alkyl which is substituted once or twice, independently of one another, by -OH, $NH_2$, -COOH, -$CONH_2$, -NH-C($NH_2$)=NH, $C_5$-$C_8$-cycloalkyl, a radical $R^3$ where

$R^3$ is $C_6$-$C_{14}$-aryl which can optionally be substituted by hydroxyl, is a bicyclic 8- to 12-membered heterocyclic aromatic ring which contains as hetero element a nitrogen atom, or a radical $R^4$

where

$R^4$ is $NR^5R^6$, a residue of a natural or unnatural amino acid, imino acid, optionally N-$C_1$-$C_4$-alkylated or N-$C_6$-$C_{14}$-aryl-$C_1$-$C_4$-alkylated azaamino acid or a dipeptide, and the amides thereof, or a radical $COR^{4'}$ in which $R^{4'}$ is defined as $R^4$,

$R^5$ is hydrogen,

$R^6$ is $C_1$-$C_6$-alkyl, and

n is 3.

**6.** A process for preparing a compound of the formula I as claimed in claims 1 to 4, which comprises

$a_1$) heating compounds of the formula IV

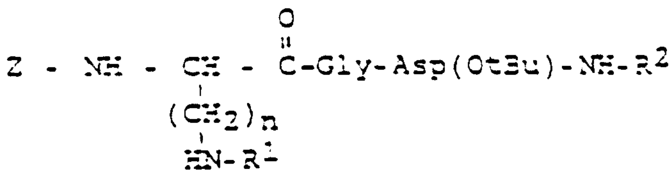 (IV),

in which Z, $R^1$, $R^2$ and n have the meanings indicated in claims 1 to 5, in tetrahydrofuran under reflux for about 2-3 hours, or

$a_2$) condensing compounds of the formula VI and VII

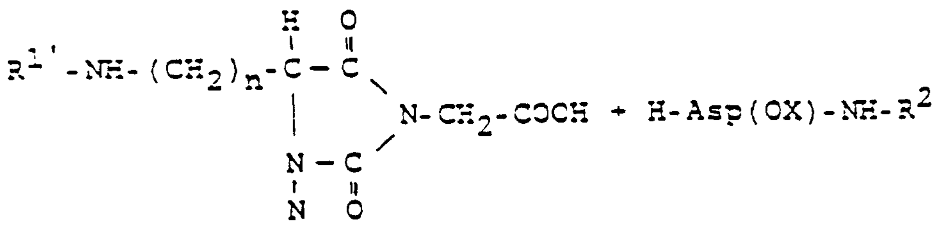

(VI) (VII)

in which

$R^{1'}$ is $-C(NH_2)=N-NO_2$ or is defined as $R^1$ above,

X is tBu or Bzl, and $R^2$ and n have the meanings described above, by general methods of peptide chemistry, and

b) converting the hydantoins of the general formula V which have been produced in this way

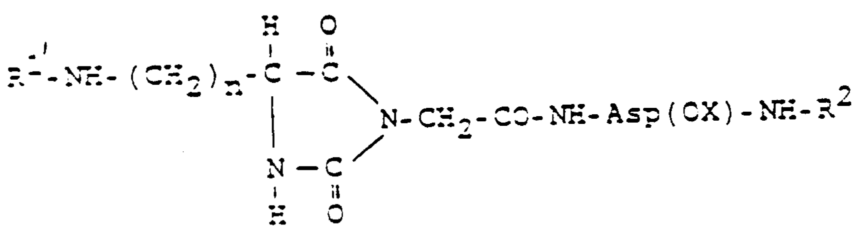 (V)

by catalytic hydrogenation and/or acid elimination of the protective groups into the compounds of the formula I according to the invention.

**7.** A compound of the formula I as claimed in claims 1 to 5 for use as medicine.

**8.** A compound of the formula I as claimed in claims 1 to 5 for inhibiting platelet aggregation.

**9.** A compound of the formula I as claimed in claims 1 to 5 for inhibiting the binding of osteoclasts to the surface of bone.

**10.** A pharmaceutical composition containing a compound of the formula I as claimed in claims 1 to 5 or the physiologically tolerated salt thereof and a physiologically acceptable vehicle.

**Claims for the following Contracting States : ES, GR**

**1.** Process for preparing a compound of the formula I

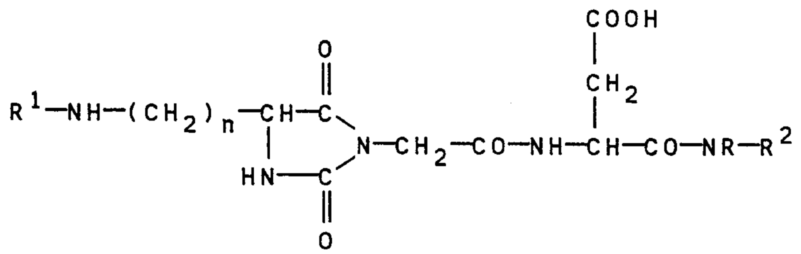

(I)

in which

n is an integer 3 or 4;

$R^1$ is $C_1$-$C_6$-alkyl or a radical of the formula II

R'-NH-C = N-R''

in which

R' and R'' are, independently of one another, hydrogen or $C_1$-$C_6$-alkyl;

$R^2$ is hydrogen, $-NH-CO-NH_2$ or $C_1$-$C_{18}$-alkyl which is optionally substituted one or more times by identical or different radicals from the series comprising hydroxyl, carbamoyl, carboxyl, carboxamido, amino, mercapto, $C_1$-$C_{18}$-alkoxy, guanidino, $C_3$-$C_8$-cycloalkyl, halogen, nitro, trifluoromethyl and a radical $R^3$, where

$R^3$ is $C_6$-$C_{14}$-aryl $C_6$-$C_{14}$-aryl-$C_1$-$C_8$-alkyl or a mono- or bicyclic 5- to 12-membered heterocyclic ring which can be aromatic, partially hydrogenated or completely hydrogenated and which can contain as hetero element one, two or three identical or different nitrogen, oxygen or sulfur atoms, where the aryl and, independently of one another, the heterocyclic radical is optionally substituted one or more times by identical or different radicals from the series comprising $C_1$-$C_{18}$-alkyl, $C_1$-$C_{18}$-alkoxy, halogen, hydroxyl, nitro and trifluoromethyl; or is a radical $R^4$; where

$R^4$ is $NR^5R^6$; $OR^5$; $SR^5$; an amino-acid side-chain; a residue of a natural or unnatural amino acid, imino acid, optionally N-$C_1$-$C_8$-alkylated or $C_6$-$C_{14}$-aryl-$C_1$-$C_8$-alkylated azaamino acid or a dipeptide in which the peptide linkage can be reduced to $NH-CH_2$, and the esters and amides thereof, where free functional groups can optionally be replaced by hydrogen or hydroxymethyl or protected by the protective groups customary in peptide chemistry; or is a radical $COR^{4'}$ in which $R^{4'}$ is defined as $R^4$;

$R^5$ is hydrogen, optionally amino group-substituted $C_1$-$C_{18}$-alkyl, $C_6$-$C_{14}$-aryl, $C_8$-$C_{14}$-aryl-$C_1$-$C_8$-alkyl, $C_1$-$C_{18}$-alkylcarbonyl, $C_1$-$C_{18}$-alkyloxycarbonyl, $C_6$-$C_{14}$-arylcarbonyl, $C_6$-$C_{12}$-aryl-$C_1$-$C_8$-alkylcarbonyl, $C_6$-$C_{18}$-aryl-$C_1$-$C_{18}$-alkyloxycarbonyl, a residue of a natural or unnatural amino acid, imino acid, optionally N-$C_1$-$C_8$-alkylated or $C_6$-$C_{14}$-aryl-$C_1$-$C_8$-alkylated azaamino acid or a dipeptide in which the peptide linkage can be reduced to $NH-CH_2$;

$R^6$ is hydrogen, $C_1$-$C_{18}$-alkyl, $C_6$-$C_{12}$-aryl or $C_6$-$C_{12}$-aryl-$C_1$-$C_8$-alkyl;

and the physiologically tolerated salts thereof, characterized in that compounds of the formula IV

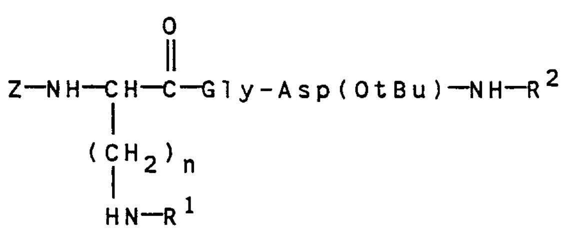

(IV),

in which $R^1$, $R^2$ and n have the meanings indicated in claims 1 to 4, are dissolved in trifluoroacetic acid and left to stand at room temperature for 30 minutes to 3 hours.

2. Process according to Claim 1, characterized in that a compound of the formula I is prepared, wherein

$R^2$ is hydrogen or $C_1$-$C_8$-alkyl which is optionally substituted 1 to 4 times by identical or different radicals from the series comprising hydroxyl, amino, carboxyl, carbamoyl, guanidino, $C_1$-$C_6$-alkoxy, $C_3$-$C_6$-cycloalkyl, halogen and a radical $R^3$, where

$R^3$ is $C_6$-$C_{12}$-aryl, $C_6$-$C_{12}$-aryl-$C_1$-$C_4$-alkyl or a mono-or bicyclic 5- to 12-membered heterocyclic aromatic ring which contains as hetero element one or two nitrogen atoms, where, independently of one another, the aryl and heterocyclic radical are optionally substituted once, twice or three times by identical or different radicals from the series comprising $C_1$-$C_6$-alkyl, $C_1$-$C_6$-alkoxy, halogen, nitro, hydroxyl and trifluoromethyl; or is a radical $R^4$, where

$R^4$ is $NR^5R^6$; $OR^5$; an amino-acid side-chain; a residue of a natural or unnatural amino acid, imino acid, optionally N-$C_1$-$C_8$-alkylated or $C_6$-$C_{14}$-aryl-$C_1$-$C_8$-alkylated azaamino acid or a dipeptide, and the esters and amides thereof, where free functional groups can optionally be replaced by hydrogen or hydroxymethyl or protected by protective groups customary in peptide chemistry; or is a radical $COR^{4'}$ in which $R^{4'}$ is defined as $R^4$;

$R^5$ and $R^6$ are, independently of one another, hydrogen, $C_1$-$C_8$-alkyl or $C_6$-$C_{12}$-aryl.

3. Process according to Claims 1 and 2, characterized in that a compound of the formula I is prepared, wherein

$R^1$ is a radical of the formula II in which

R' and R'' are, independently of one another, hydrogen or $C_1$-$C_2$-alkyl;

$R^2$ is hydrogen or $C_1$-$C_6$-alkyl which is substituted twice by identical or different radicals $R^4$, where $R^4$ is hydroxyl; amino; an amino-acid side-chain; a residue of a natural or unnatural amino acid, imino acid, optionally N-$C_1$-$C_8$-alkylated or $C_6$-$C_{14}$-aryl-$C_1$-$C_8$-alkylated azaamino acid or a dipeptide in which the peptide linkage can be reduced to $NH$-$CH_2$, and the esters and amides thereof, where free functional groups can optionally be replaced by hydrogen or hydroxymethyl or protected by protective groups customary in peptide chemistry; or is a radical $COR^{4'}$ in which $R^{4'}$ is defined as $R^4$.

4. Process according to Claims 1 to 3, characterized in that a compound of the formula I is prepared, wherein

$R^2$ is methyl which is subtituted twice by different radicals $R^4$ and $COR^{4'}$ where $R^4$ and $R^{4'}$ are defined as in claim 3.

5. Process according to Claims 1 to 4, characterized in that a compound of the formula I is prepared, wherein

$R^1$ is a radical of the formula II in which R' and R'' are hydrogen,

$R^2$ is hydrogen or $C_1$-$C_6$-alkyl which is substituted once or twice, independently if one another, by -OH, $NH_2$, -COOH, -$CONH_2$, -NH-C($NH_2$)=NH, $C_5$-$C_8$-cycloalkyl, a radical $R^3$ where

$R^3$ is $C_6$-$C_{14}$-aryl which can optionally be substituted by hydroxyl, is a bicyclic 8- to 12-membered heterocyclic aromatic ring which contains as hetero element a nitrogen atom, or a radical $R^4$

where

$R^4$ is $NR^5R^5$, a residue of a natural or unnatural amino acid, imino acid, optionally N-$C_1$-$C_4$-alkylated or N-$C_6$-$C_{14}$-aryl-$C_1$-$C_4$-alkylated azaamino acid or a dipeptide, and the amides thereof, or a radical $COR^{4'}$ in which $R^{4'}$ is defined as $R^4$,

$R^5$ is hydrogen,

$R^6$ is $C_1$-$C_6$-alkyl, and

n is 3.

6. Process for preparing a pharmaceutical composition containing a compound of the formula I as claimed in claims 1 to 5 characterized in that said composition and a physiologically acceptable vehicle are converted into a form suitable for administration.

## Revendications

## Revendications pour les Etats contractants suivants : BE, DE, DK, FR, GB, IT, LU, NL

**1.** Composés de formule générale I

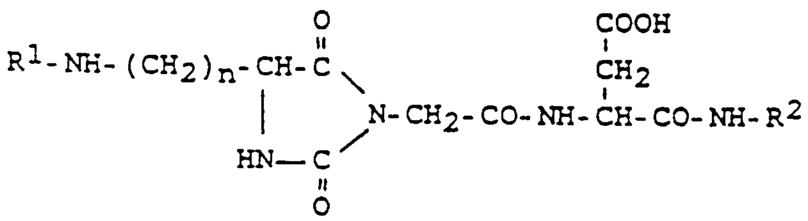

(I),

dans laquelle

n représente un des nombres entiers 3 ou 4 ;

$R^1$ signifie un alkyle en $C_1$-$C_6$ ou un reste de formule II

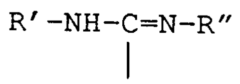

(II),

dans laquelle

R' et R'' signifient, indépendamment l'un de l'autre, un hydrogène ou un alkyle en $C_1$-$C_6$ ;

$R^2$ signifie un hydrogène, un -NH-CO-$NH_2$ ou un alkyle en $C_1$-$C_{18}$, qui est éventuellement substitué une ou plusieurs fois par des restes identiques ou différents appartenant à la série des hydroxy, carbamoyle, carboxy, carboxamido, amino, mercapto, alcoxy en $C_1$-$C_{18}$, guanidino, cycloalkyle en $C_3$-$C_8$, halogène, nitro, trifluorométhyle et un reste $R^3$,

$R^3$ représentant un aryle en $C_6$-$C_{14}$, un (aryl en $C_6$-$C_{14}$)-alkyle en $C_1$-$C_8$ ou bien un système hétérocyclique, mono- ou bicyclique, ayant de 5 à 12 chaînons, qui peut être aromatique, partiellement hydrogéné ou bien totalement hydrogéné et qui peut contenir en tant qu'hétéro-élément un, deux ou trois atomes, identiques ou différents, d'azote, d'oxygène et de soufre, le reste aryle et le reste hétérocyclique, indépendamment l'un de l'autre, étant éventuellement substitués une ou plusieurs fois par des restes identiques ou différents appartenant à la série des alkyles en $C_1$-$C_{18}$, alcoxy en $C_1$-$C_{18}$, halogène, hydroxy, nitro et trifluorométhyle ; ou bien représentant un reste $R^4$ ;

$R^4$ signifiant un $NR^5R^6$ ; un $OR^5$ ; un $SR^5$ ; une chaîne latérale d'aminoacide ; un reste de dipeptide, dans lequel la liaison peptidique peut être réduite en NH-$CH_2$, un reste d'azaaminoacide éventuellement N-alkylé par des alkyles en $C_1$-$C_8$ ou bien (aryle en $C_6$-$C_{14}$)-alkylé par des alkyles en $C_1$-$C_8$, un reste d'iminoacide ou bien un reste d'aminoacide, naturel ou non naturel, ainsi que leurs esters et leurs amides, les groupes fonctionnels libres pouvant être éventuellement substitués par des hydrogène ou hydroxyméthyle ou bien pouvant être protégés par des groupes protecteurs classiques en chimie peptidique ; ou bien signifiant un reste $COR^{4'}$, dans lequel $R^{4'}$ est défini comme pour $R^4$ ;

$R^5$ signifie un hydrogène, un reste dipeptide, dans lequel la liaison peptidique peut être réduite en NH-$CH_2$, un reste azaaminoacide éventuellement N-alkylé par des alkyles en $C_1$-$C_8$ ou bien (aryl en $C_6$-$C_{14}$)-alkylé par des alkyles en $C_1$-$C_8$, un reste d'iminoacide, un reste d'aminoacide, naturel ou non naturel, un (aryl en $C_6$-$C_{18}$)-(alkyl en $C_1$-$C_{18}$)-oxycarbonyle, un (aryl en $C_6$-$C_{12}$)-(alkyl en $C_1$-$C_8$)-carbonyle, un (aryl en $C_6$-$C_{14}$)-carbonyle, un (alkyl en $C_1$-$C_{18}$)-oxycarbonyle, un (alkyl en $C_1$-$C_{18}$) - carbonyle, un (aryl en $C_6$-$C_{14}$)-alkyle en $C_1$-$C_8$, un aryle en $C_6$-$C_{14}$ ou bien un alkyle en $C_1$-$C_{18}$, éventuellement substitué par un groupe amino ;

$R^6$ signifie un hydrogène, un alkyle en $C_1$-$C_{18}$, un aryle en $C_6$-$C_{12}$ ou bien un (aryl en $C_6$-$C_{12}$)-alkyle en $C_1$-$C_8$ ;

ainsi que leurs sels compatibles du point de vue physiologique.

**2.** Composés de formule (I) selon la revendication 1, caractérisés en ce que

$R^2$ signifie un hydrogène ou un alkyle en $C_1$-$C_8$, qui est éventuellement substitué de une à quatre fois par des restes identiques ou différents appartenant à la série des hydroxy, amino, carboxy, carbamoyle, guanidino, alcoxy en $C_1$-$C_6$, cycloalkyle en $C_3$-$C_6$, halogène et un reste $R^3$,

$R^3$ représentant un aryle en $C_6$-$C_{12}$, un (aryl en $C_6$-$C_{12}$)-alkyle en $C_1$-$C_4$ ou bien un système cyclique aromatique, hétérocyclique, mono ou bicyclique, ayant de 5 à 12 chaînons, qui contient, en tant qu'hétéro-élément, un ou deux atomes d'azote, le reste aryle et le reste hétérocyclique, indépendamment l'un de l'autre, étant éventuellement substitués une, deux ou trois fois par des restes identiques ou différents appartenant à la série des alkyles en $C_1$-$C_6$, alcoxy en $C_1$-$C_6$, halogène, nitro, hydroxy et trifluorométhyle ; ou bien représentant un reste $R^4$,

$R^4$ signifiant un $NR^5R^6$ ; un $OR^5$ ; une chaîne latérale d'aminoacide ; un reste un reste de dipeptide, d'azaaminoacide éventuellement N-alkylé par des alkyles en $C_1$-$C_8$ ou bien (aryle en $C_6$-$C_{14}$)-alkylé par des alkyles en $C_1$-$C_8$, d'un iminoacide, d'un aminoacide, naturel ou non naturel, ainsi que leurs esters et leurs amides, les groupes fonctionnels libres pouvant être éventuellement substitués par des hydrogène ou hydroxyméthyle ou bien pouvant être protégés par des groupements protecteurs classiques en chimie peptidique ; ou bien un reste $COR^{4'}$, dans lequel $R^{4'}$ est défini comme pour $R^4$ ;

$R^5$ et $R^6$ signifient, indépendamment l'un de l'autre, un hydrogène, un alkyle en $C_1$-$C_8$ ou un aryle en $C_6$-$C_{12}$.

**3.** Composés de formule (I) selon les revendications 1 à 2, caractérisés en ce que

$R^1$ signifie un reste de formule (II), dans laquelle

R' et R'' signifient, indépendamment l'un de l'autre, un hydrogène ou un alkyle en $C_1$-$C_2$ ;

$R^2$ signifie un hydrogène ou un alkyle en $C_1$-$C_6$, qui est substitué deux fois par des restes $R^4$ identiques ou différents,

$R^4$ représentant un hydroxy ; un amino ; une chaîne latérale d'un aminoacide ; , un reste d'un iminoacide, d'un azaaminoacide éventuellement N-alkylé par des alkyles en $C_1$-$C_8$ ou bien (aryl en $C_6$-$C_{14}$)-alkylé par des alkyles en $C_1$-$C_8$, d'un dipeptide, dont la liaison peptidique peut être réduite en $NH$-$CH_2$, ou d'un aminoacide, naturel ou non naturel, ainsi que leurs esters et leurs amides, les groupes fonctionnels libres pouvant être substitués éventuellement par des hydrogène ou hydroxyméthyle ou bien pouvant être protégés par des groupements protecteurs classiques dans la chimie peptidique ; ou bien un reste $COR^{4'}$ dans lequel $R^{4'}$ est tel que défini pour $R^4$.

**4.** Composés de formule I selon les revendications 1 à 3, caractérisés en ce que

$R^2$ signifie un méthyle, qui est substitué deux fois par différents restes $R^4$ et $COR^{4'}$, $R^4$ ou bien $R^{4'}$ étant défini comme dans la revendication 3.

**5.** Composés de formule I, selon les revendications 1 à 4, caractérisés en ce que

$R^1$ signifie un reste de formule II dans laquelle
R' et R'' signifient un hydrogène,

$R^2$ signifie un hydrogène ou un alkyle en $C_1$-$C_6$, qui est substitué une ou deux fois, indépendamment l'un de l'autre, par des -OH, $NH_2$, -COOH, $-CONH_2$, $-NH-C(NH_2)=NH$, cycloalkyle en $C_5$-$C_8$, un reste $R^3$,

$R^3$ représentant un aryle en $C_6$-$C_{14}$, qui peut être substitué éventuellement par des hydroxy, un système cyclique aromatique, hétérocyclique, de 8 à 12 chaînons, bicyclique, qui contient comme, hétéroélément, un atome d'azote,
ou bien un reste $R^4$,

$R^4$ représentant un $NR^5R^6$, un reste d'iminoacide, d'azaaminoacide éventuellement N-alkylé par des alkyles en $C_1$-$C_4$ ou bien N-(aryle en $C_6$-$C_{14}$)-alkylé par des alkyles en $C_1$-$C_4$, d'un dipeptide ou d'un aminoacide, naturel ou non naturel, ainsi que leurs amides, ou bien un reste $COR^{4'}$, dans lequel $R^{4'}$ est défini comme pour $R^4$,

$R^5$ signifie un hydrogène,

$R^6$ signifie un alkyle en $C_1$-$C_6$, et

n signifie 3.

6. Procédé de préparation d'un composé de formule I selon les revendications 1 à 4, caractérisé en ce que

a₁) on porte au reflux des composés de formule IV,

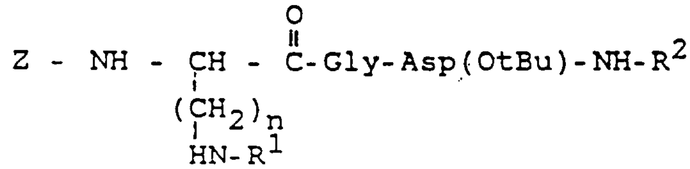

(IV),

dans laquelle Z, $R^1$, $R^2$ et n ont les significations indiquées dans les revendications 1 à 5, dans du tétrahydrofuranne pendant 2 à 3 heures ou bien

a₂) on condense les composés de formules générales VI et VII

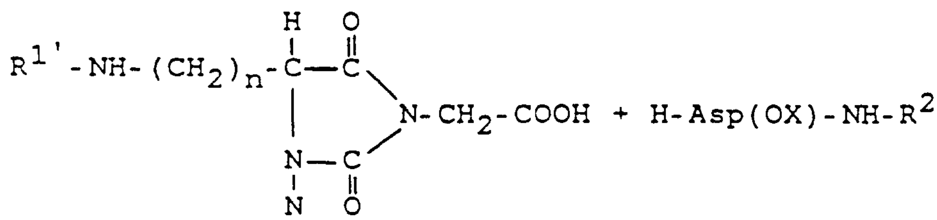

(VI) (VII)

dans lesquelles

$R^{1'}$ signifie $-C(NH_2)=N-NO_2$ ou bien est défini comme pour $R^1$ ci-dessus,

X représente tBu ou bien Bzl, $R^2$ et n ont les significations décrites ci-dessus,

au moyen de méthodes générales en chimie peptidique, et

b) on transforme les hydantoïnes ainsi formées de formule générale V :

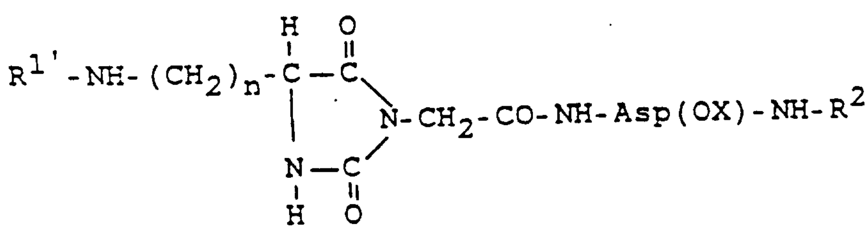

(V)

par hydrogénation catalytique et/ou élimination en milieu acide des groupements protecteurs en les composés de formule générale I conformes à l'invention.

7. Composé de formule I selon les revendications 1 à 5 en vue d'une utilisation en tant que médicament.

8. Composé de formule I selon les revendications 1 à 5 afin d'inhiber l'agrégation des thrombocytes.

9. Composé de formule I selon les revendications 1 à 5 afin d'inhiber la liaison d'ostéoclastes sur la surface osseuse.

10. Préparation pharmaceutique contenant un composé de formule I selon les revendications 1 à 5 ou un de ses sels compatibles du point de vue physiologique et un support acceptable du point de vue physiologique.

**Revendications pour les Etats contractants suivants : ES, GR**

**1.** Procédé de prépration d'un composé de formule générals I

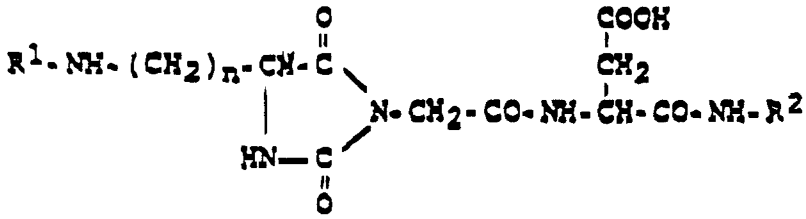

(I),

dans laquelle

n représente un des nombres entiers 3 ou 4 ;

$R^1$ signifie un alkyle on $C_1$-$C_6$ ou un reste de formule II

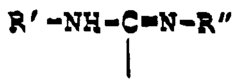

(II),

dans laquelle

R' et R" signifient, indépendamment l'un de l'autre, un hydrogène ou un alkyle en $C_1$-$C_6$ ;

$R^2$ signifie un hydrogène, un -NH-CO-$NH_2$ ou un alkyle en $C_1$-$C_{18}$, qui est éventuellement substitué une ou plusieurs fois par des restes identiques ou différents appartenant à la série des hydroxy, carbamoyle, carboxy, carboxamido, amino, mercapto, alcoxy on $C_1$-$C_{18}$, guanidino, cycloalkyle en $C_3$-$C_8$, halogène, nitro, trifluorométhyle et un reste $R^3$,

$R^3$ représentant un aryle en $C_6$-$C_{14}$, un (aryl en $C_6$-$C_{14}$)-alkyle en $C_1$-$C_8$ ou bien un système hétérocyclique, mono- ou bicyclique, ayant de 5 à 12 chaînons, qui peut être aromatique, partiellement hydrogéné ou bien totalement hydrogéné et qui peut contenir en tant qu'hétéro-élément un, deux ou trois atomes, identiques ou différents, d'azote, d'oxygène et de soufre, le reste aryle et le reste hétérocyclique, indépendamment l'un de l'autre, étant éventuellement substitués une ou plusieurs fois par des restes identiques ou différents appartenant à la série des alkyles en $C_1$-$C_{18}$, alcoxy en $C_1$-$C_{18}$, halogène, hydroxy, nitro et trifluorométhyle ; ou bien représentant un reste $R^4$ ;

$R^4$ signifiant un $NR^5R^6$ ; un $OR^5$ ; un $SR^5$ ; une chaîne latérale d'aminoacide ; un reste de dipeptide, dans lequel la liaison peptidique peut être réduite en $NH$-$CH_2$, un reste d'azaaminoacide éventuellement N-alkylé par des alkyles on $C_1$-$C_8$ ou bien (aryle en $C_6$-$C_{14}$)-alkylé par des alkyles en $C_1$-$C_8$, un reste d'iminoacide ou bien un reste d'aminoacide, naturel ou non naturel, ainsi que leurs esters et leurs amides, les groupes fonctionnels libres pouvant être éventuellement substitués par des hydrogène ou hydroxyméthyle ou bien pouvant être protégés par des groupes protecteurs classiques on chimie peptidique ; ou bien signifiant un reste $COR^{4'}$, dans lequel $R^{4'}$ est défini comme pour $R^4$ ;

$R^5$ signifie un hydrogène, un reste dipeptide, dans lequel la liaison peptidique peut être réduite en $NH$-$CH_2$, un reste azaaminoacide éventuellement N-alkylé par des alkyles en $C_1$-$C_8$ ou bien (aryl en $C_6$-$C_{14}$)-alkylé par des alkyles en $C_1$-$C_8$, un reste d'iminoacide, un reste d'aminoacide, naturel ou non naturel, un (aryl en $C_6$-$C_{18}$)-(alkyl en $C_1$-$C_{18}$)-oxycarbonyle, un (aryl en $C_6$-$C_{12}$)-(alkyl) en $C_1$-$C_8$)-carbonyle, un (aryl en $C_6$-$C_{14}$)-carbonyle, un (alkyl en $C_1$-$C_{18}$)-oxycarbonyle, un (alkyl en $C_1$-$C_{18}$)-carbonyle, un (aryl en $C_6$-$C_{14}$)-alkyle en $C_1$-$C_8$, un aryle en $C_6$-$C_{14}$ ou bien un alkyle en $C_1$-$C_{18}$, éventuellement substitué par un groupe amino ;

$R^6$ signifie un hydrogène, un alkyle en $C_1$-$C_{18}$, un aryle en $C_6$-$C_{12}$ ou bien un (aryl en $C_6$-$C_{12}$)-alkyle an $C_1$-$C_8$ ;

ainsi que ses sels compatibles du point de vue physiologique, caractérisé on ce qu'on dissout dans de l'acide trifluoroacétique des composés de formule IV

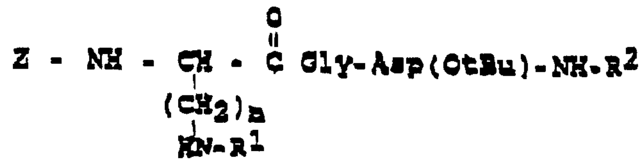 (IV),

dans laquelle $R^1$, $R^2$ et n ont les significations données dans les revendications 1 à 4, et on les laisse à la température ambiance pendant 30 minutes à 3 heures.

**2.** Procédé selon la revendication 1, caractérisé en ce qu'on prépare un composé de formule I, dans laquelle

$R^2$ signifie un hydrogène ou un alkyle en $C_1$-$C_8$, qui est éventuellement substitué de une à quatre fois par des restes identiques ou différents appartenant à la série des hydroxy, amino, carboxy, carbamoyle, guanidino, alcoxy en $C_1$-$C_6$, cycloalkyle en $C_3$-$C_6$, halogène et un reste $R^3$,

$R^3$ représentant un aryle on $C_6$-$C_{12}$, un (aryl an $C_6$-$C_{12}$)-alkyle en $C_1$-$C_4$ ou bien un système cyclique aromatique, hétérocyclique, mono ou bicyclique, ayant de 5 à 12 chaînons, qui contient, en tant qu'hétéro-élément, un ou deux atomes d'azote, le reste aryle et le rente hétérocyclique, indépendamment l'un de l'autre, étant éventuellement substitués une, deux ou trois fois par des restes identiques ou différents appartenant à la série des alkyles on $C_1$-$C_6$, alcoxy en $C_1$-$C_6$, halogène, nitro, hydroxy et trifluorométhyle ; ou bien représentant un reste $R^4$,

$R^4$ signifiant un $NR^5R^6$ ; un $OR^5$ ; une chaîne latérale d'aminoacide ; un reste un reste de dipeptide, d'azaaminoacide éventuellement N-alkylé par des alkyles en $C_1$-$C_8$ ou bien (aryle en $C_6$-$C_{14}$)-alkylé par des alkyles en $C_1$-$C_8$, d'un iminoacide, d'un aminoacide, naturel ou non naturel, ainsi que leurs esters et leurs amides, les groupes fonctionneis libres pouvant être éventuellement substitués par des hydrogène ou hydroxyméthyle ou bien pouvant être protégés par des groupements protecteurs classiques en chimie peptidique ; ou bien un reste $COR^{4'}$, dans lequel $R^{4'}$ est défini comme pour $R^4$ ;

$R^5$ et $R^6$ signifient, indépendamment l'un de l'autre, un hydrogène, un alkyle en $C_1$-$C_8$ ou un aryle en $C_6$-$C_{12}$.

**3.** Procédé selon les revendications 1 et 2, caractérisé en ce qu'on prépare un composé de formule I, dans laquelle

$R^1$ signifie un reste de formule (II), dans laquelle

R' et R'' signifient, indépendamment l'un de l'autre, un hydrogène ou un alkyle un $C_1$-$C_2$ ;

$R^2$ signifie un hydrogène ou un alkyle en $C_1$-$C_6$, qui est substitué deux fois par des restes $R^4$ identiques ou différents,

$R^4$ représentant un hydroxy ; un amino ; une chaîne latérale d'un aminoacide ; , un reste d'un iminoacide, d'un azaaminoacide éventuellement N-alkylé par don alkyles on $C_1$-$C_8$ ou bien (aryl en $C_6$-$C_{14}$)-alkylé par des alkyles en $C_1$-$C_8$, d'un dipeptide, dont la liaison peptidique peut être réduite en $NH$-$CH_2$, ou d'un aminoacide, naturel ou non naturel, ainsi que leurs esters et leurs amides, les groupes fonctionnels libres pouvant être substitués éventuellement par des hydrogène ou hydroxyméthyle ou bien pouvant être protégés par des groupements protecteurs classiques dans la chimie peptidique ; ou bien un reste $COR^{4'}$ dans lequel $R^{4'}$ est tel que défini pour $R^4$.

**4.** Procédé salon les revendications 1 à 3, caractérisé en ce qu'on prépare un compose de formule I, dans laquelle

$R^2$ signifie un méthyle, qui est substitué deux fois par différents restes $R^4$ et $COR^{4'}$, $R^4$ ou bien $R^{4'}$, étant défini comme dans la revendication 3.

**5.** Procédé selon les revendications 1 à 4, caractérisé en ce qu'on prépare un composé de formule I, dans laquelle

$R^1$ signifie un reste de formule II, dans laquelle

R' et R'' signifient un hydrogène,

$R^2$ signifie un hydrogène ou un alkyle en $C_1$-$C_6$, qui est substitué une ou deux fois, indépendamment l'un de l'autre, par des -OH, $NH_2$, -COOH, -$CONH_2$, -NH-C($NH_2$) = NH, cycloalkyle en $C_5$-$C_8$, un reste $R^3$,

$R^3$ représentant un aryle en $C_6$-$C_{14}$, qui peut être substitué éventuellement par des hydroxy, un système cyclique aromatique, hétérocyclique, de 8 à 12 chaînons, bicyclique, qui contient comme, hétéro-élément, un atome d'azote,
ou bien un reste $R^4$,

$R^4$ représentant un $NR^5R^6$, un reste d'iminoacide, d'azaaminoacide éventuellement N-alkylé par des alkyles en $C_1$-$C_4$ ou bien N-(aryle on $C_6$-$C_{14}$)-alkylé par des alkyles en $C_1$-$C_4$, d'un dipeptide ou d'un aminoacide, naturel ou non naturel, ainsi que leurs amides, ou bien un reste $COR^{4'}$, dans lequel $R^{4'}$ est défini comme pour $R^4$,

$R^5$ signifie un hydrogène,

$R^6$ signifie un alkyle en $C_1$-$C_6$, et

n signifie 3.

**6.** Procédé de préparation d'une préparation pharmaceutique contenant un composé de formule I selon les revendications 1 à 5, caractérisé en ce qu'on met sous une forme de préparation appropriée ce composé et un support acceptable du point de vue physiologique.